# EUROPEAN PATENT APPLICATION

(11) **EP 3 804 811 A1**
(43) Date of publication of application: **14.04.2021**
(21) Application number: 20194346.1
(22) Date of filing: 13.05.2014
(51) Int. Cl.: A61P 25/24, A61K 45/06, A61K 38/22

(54) **TREATMENT OF MOOD AND ANXIETY DISORDERS**

(30) Priority: 13.05.2013 US 201361822619 P
(62) Divisional of application: 14798340.7
(71) Applicant: Icahn School of Medicine at Mount Sinai, New York, NY 10029 (US)
(72) Inventor: CHARNEY, Dennis S., Chappaqua, NY New York 10514 (US); MURROUGH, James, Mamaroneck, NY New York (US)
(74) Representative: Grünecker Patent- und Rechtsanwälte PartG mbB

(57) **Abstract**

The present disclosure provides compositions and formulations containing neuropeptide Y and methods of their use for the treatment of mood and anxiety disorders.

## Description

### CROSS-REFERENCE TO RELATED APPLICATION

This application claims the benefit of priority under 35 U.S.C. § 119(e) to provisional U.S. Patent Application No. 61/822,619, filed May 13, 2013.

### TECHNICAL FIELD

This disclosure relates to methods and compositions for treatment of mood and anxiety disorders.

### BACKGROUND

Mood disorders, such as, e.g., Major Depressive Disorder and bipolar disorder, and anxiety disorders, such as, e.g., panic disorder, obsessive-compulsive disorder (OCD), post-traumatic stress disorder (PTSD), generalized anxiety disorder, and phobias (social phobia and specific phobia), are prevalent and often difficult to treat. Approximately 20.9 million American adults, or about 9.5 percent of the U.S. population age 18 and older in a given year, have a mood disorder. Depressive disorders often co-occur with anxiety disorders and substance abuse. Major Depressive Disorder is the leading cause of disability in the U.S. for ages 15-44. Major depressive disorder affects approximately 14.8 million American adults, or about 6.7 percent of the U.S. population age 18 and older in a given year. While major depressive disorder can develop at any age, the median age at onset is 32.5. Major depressive disorder is more prevalent in women than in men. Approximately 40 million American adults ages 18 and older, or about 18.1 percent of people in this age group in a given year, have an anxiety disorder. Anxiety disorders frequently co-occur with depressive disorders or substance abuse. Most people with one anxiety disorder also have another anxiety disorder. Nearly three-quarters of those with an anxiety disorder will have their first episode by age 21.5.

PTSD, for example, is a highly debilitating psychiatric disorder that is notoriously difficult to treat. PTSD is characterized by flashbacks, emotional numbness, and insomnia, and is associated with functional impairments, physical health concerns, and mental health comorbidities, such as depression, with six fold higher risk of suicide. It can result from a catastrophic and threatening event such as a natural disaster, wartime situation, accident, domestic abuse, or violent crime. Symptoms usually develop within three months, but can emerge years after the initial trauma. At some point in their lifetimes, 5-8% of men, 10-14% of women, and up to 25% of combat veterans are diagnosed with PTSD. The treatment of PTSD is extremely challenging, and may include many years of individual and group therapy, and medications such as antidepressants, anxiolytic drugs, β-adrenergic antagonists, opiates, or cortisol, with variable results. Selective serotonin reuptake inhibitors (SSRIs) are currently recommended as the first-line pharmacotherapy. However, up to 40% of SSRI-treated PTSD patients do not respond and >70% never achieve full remission. The two SSRIs that are approved for PTSD by the United States (US) Food and Drug Administration (FDA), paroxetine and sertraline, have modest effect sizes and limited efficacy in all three clusters of illness: re-experiencing, avoidance and numbing, and hyperarousal. In fact, in 2007, the Institute of Medicine issued a report on the treatment of PTSD and found that current treatments lack a high level of scientific evidence to support their effectiveness.

PTSD is particularly prevalent among combat veterans. An estimated 17% of Operation Iraqi Freedom/Operation Enduring Freedom veterans will develop PTSD. A recent Veterans Affairs (VA) clinical trial of the FDA-approved drug, sertraline, failed to show efficacy in a group of patients with predominantly combat-related PTSD. The severity and significance of lack of SSRI efficacy, especially in light of the observed relationship between trauma exposure and increased rates of disability, unemployment, and social assistance highlights the urgent need for novel pharmacological interventions targeting the core pathophysiology of PTSD.

There is a need in the art for improved methods for treating mood and anxiety disorders.

### SUMMARY

As follows from the Background section, above, there is a need in the art for improved methods for the treatment of mood and/or anxiety disorders. The present disclosure provides therapeutic agents and methods for treating mood (e.g., Major Depressive Disorder) and anxiety disorders (e.g., OCD, PTSD).

In certain aspects, the present disclosure provides a method for treating a human patient for a mood or anxiety disorder, which comprises intranasally administering to a human patient in need of such treatment a composition comprising a therapeutically effective amount of neuropeptide Y (NPY) for reducing or eliminating the symptoms (or one more of the symptoms) of the mood or anxiety disorder, wherein the therapeutically effective amount is a dosage range of about 0.005 milligrams per kilogram of body weight of the patient (mg/kg) to about 1 mg/kg. In further aspects, a method for treating a human patient for a mood or anxiety disorder is provided which comprises intranasally administering to a human patient in need of such treatment a composition comprising a therapeutically effective amount of a Y1 receptor agonist for reducing or eliminating the symptoms of the mood or anxiety disorder, wherein the therapeutically effective amount is a dosage range of about 0.005 milligrams per kilogram of body weight of the patient (mg/kg) to about 1 mg/kg. In other aspects, a method for treating a human patient for a mood or anxiety disorder is provided which comprises intranasally administering to a human patient in need of such treatment a composition comprising a therapeutically effective amount of a Y2 receptor antagonist for reducing or eliminating the symptoms of the mood or anxiety disorder, wherein the therapeutically effective amount is a dosage range of about 0.005 milligrams per kilogram of body weight of the patient (mg/kg) to about 1 mg/kg.

In the above aspects, the therapeutically effective amount can be a dosage range of about 0.005 mg/kg to about 0.75 mg/kg, about 0.01 mg/kg to about 0.75 mg/kg, 0.05 mg/kg to about 0.75 mg/kg, 0.05 mg/kg to about 0.5 mg/kg, 0.01 mg/kg to about 0.5 mg/kg, 0.01 mg/kg to about 0.25 mg/kg, or 0.1 mg/kg to about 0.5 mg/kg. The therapeutically effective amount can be a dosage of about 0.05 mg/kg, or a dosage of about 1.0 mg/kg, or a dosage of about 0.5 mg/kg.

In some aspects, provided herein is a method for treating a human patient for a mood or anxiety disorder which includes intranasally administering to a human patient in need of such treatment a composition comprising a therapeutically effective dose of neuropeptide Y (NPY) for reducing or eliminating one or more symptoms of the mood or anxiety disorder. In some aspects, the therapeutically effective dose is in the range of about 1 mg to about 20 mg of NPY. In some aspects, the method includes administering the therapeutically effective dose to the patient at least once a day. In some aspects, the dose is in the range of about 1 mg to about 100 mg, about 1 mg to about 90 mg, about 1 mg to about 80 mg, about 1 mg to about 70 mg, about 1 mg to about 60 mg, about 1 mg to about 50 mg, about 1 mg to about 40 mg, about 1 mg to about 30 mg, about 1 mg to about 20 mg, about 1 mg to about 10 mg, or about 1 mg to about 5 mg. In some aspects, the method includes administering a total dose to the patient of between about 1 and about 100 mg/day of NPY. In some aspects, the total dose is in the range of about 1 mg to about 100 mg, about 1 mg to about 90 mg, about 1 mg to about 80 mg, about 1 mg to about 70 mg, about 1 mg to about 60 mg, about 1 mg to about 50 mg, about 1 mg to about 40 mg, about 1 mg to about 30 mg, about 1 mg to about 20 mg, about 1 mg to about 10 mg, or about 1 mg to about 5 mg. In some aspects, the method includes administering to the subject a single dose of the composition. In some aspects, the method includes administering to the subject multiple doses of the composition. In some aspects, the method includes administering to the subject from 1 to 4 doses of the composition per day.

In any of the above, aspects, the method can include administering a single dose of the composition or multiple doses of the composition.

In some aspects of the above methods, the intranasal administration comprises atomizing the dose administered to the patient. The atomized dose can be administered to the patient as one or more intranasal sprays. The multiple doses can be administered on separate days. In some aspects at least two of the multiple doses are administered on the same day.

In some aspects of the above methods, the methods include co-administering the composition and a second active agent together or at spaced-apart time intervals in a combination therapy. In some aspects, the second active agent comprises an antidepressant medication or other psychotropic medication such as a benzodiazepine. In other aspects, the second agent is ketamine. The composition can further comprise a pharmaceutically acceptable carrier. The pharmaceutically acceptable carrier can be sterile saline or water.

In any of the above methods, the disorder can be post-traumatic stress disorder (PTSD), Major Depressive Disorder, Panic Disorder, Specific phobia, Social Phobia, Obsessive-Compulsive Disorder (OCD), acute stress disorder, or generalized anxiety disorder.

In one aspect, a pharmaceutical formulation comprising: (a) at least about 0.005 mg/kg NPY and (b), a pharmaceutically acceptable carrier, wherein the pharmaceutical formulation is suitable for intranasal administration is provided. In another aspect, a pharmaceutical formulation comprising: (a) at least about 0.01 mg/kg NPY and (b), a pharmaceutically acceptable carrier, wherein the pharmaceutical formulation is suitable for intranasal administration, is provided. In another aspect, a pharmaceutical formulation comprising: (a) at least about 0.05 mg/kg NPY and (b), a pharmaceutically acceptable carrier, wherein the pharmaceutical formulation is suitable for intranasal administration, is provided.

A method of treating a mood or anxiety disorder is provided comprising administering to a human patient in need of such treatment the pharmaceutical formulation comprising administering a pharmaceutical formulation comprising: (a) at least about 0.005 mg/kg NPY and (b), a pharmaceutically acceptable carrier, wherein the pharmaceutical formulation is suitable for intranasal administration. In another aspect, a method of treating a mood or anxiety disorder is provided comprising administering a pharmaceutical formulation comprising: (a) at least about 0.01 mg/kg NPY and (b), a pharmaceutically acceptable carrier, wherein the pharmaceutical formulation is suitable for intranasal administration. In another aspect, a method of treating a mood or anxiety disorder is provided comprising administering a pharmaceutical formulation comprising: (a) at least about 0.05 mg/kg NPY and (b), a pharmaceutically acceptable carrier, wherein the pharmaceutical formulation is suitable for intranasal administration. In the above aspects, the mood or anxiety disorder can be PTSD, Major Depressive Disorder, Panic Disorder, Specific phobia, Social Phobia, Obsessive-Compulsive Disorder (OCD), acute stress disorder, or generalized anxiety disorder.

Also provided herein is an intranasal dosage forms containing NPY and a pharmaceutically acceptable diluent. For example, the dosage forms can include NPY in a dosage range from about 0.005 milligrams per kilogram of body weight (mg/kg) to about 2 mg/kg, from about 0.005 mg/kg to about 1.5 mg/kg, from about 0.005 mg/kg to about 1 mg/kg, from about 0.01 mg/kg to about 1.0 mg/kg, from about 0.05 mg/kg to about 1.0 mg/kg, or from about 0.05 mg/kg to about 0.5 mg/kg. In another aspect, the dosage forms can include NPY in a dosage range from about 0.05 mg to about 200 mg, from about 0.05 mg to about 150 mg, from about 0.05 mg to about 125 mg, from about 0.5 mg to about 100 mg, from about 1 mg to about 100 mg, from about 1 mg to about 50 mg, from about 1 mg to about 40 mg, from about 1 mg to about 35 mg, from about 1 mg to about 30 mg, from about 1 mg to about 25 mg, from about 1 mg to about 20 mg, from about 1 mg to about 15 mg, from about 1 mg to about 10 mg, from about from about 1 mg to about 5 mg, from about 2 mg to about 35 mg, from about 3 mg to about 30 mg, from about 4 mg to about 25 mg, from about 5 mg to about 20 mg, from about 6 mg to about 15 mg, from about 7 mg to about 10 mg, from about 8 mg to about 20 mg, from about 9 mg to about 20 mg, or from about 10 mg to about 20 mg. In some aspects, the diluent is a member selected from the group consisting of sterile water, saline solution, buffered saline and dextrose solution. In some aspect, the dosage form further contains a pharmaceutically acceptable excipient.

Also provided herein is an aerosol formulation comprising NPY or an NPY analog and an aerosol propellant. In some aspects, the aerosol dosage form further contains a dry powder. In some aspects, the aerosol dosage form further contains a bulking agent.

In any of the above aspects, NPY can be substituted with an analog of NPY, or a fragment of NPY or NPY analog, or another NPY receptor ligand, such as, e.g., another Y1 receptor agonist and/or Y2 receptor antagonist.

### Definitions:

The term "intranasal administration" in all its grammatical forms refers to administration of a drug through the nasal mucous membrane and through the nose-brain pathway directly into the central nervous system, brain or cerebrospinal fluid.

As used herein, the term "aerosol" refers to suspension of the therapeutic agent in the air. In particular, aerosol refers to the particulization or atomization of a formulation (e.g., a liquid or powder formulation) disclosed herein and its suspension in the air. Thus, an aerosol formulation is a formulation comprising NPY (or NPY analog, active fragment or conjugate of NPY or NPY analog, and/or other NPY receptor ligand (including, e.g., Y1 receptor agonist and/or Y2 receptor antagonist)) for intranasal administration.

Generally, "treating" or "treatment" of a state, disorder or condition includes: (1) preventing or delaying the appearance of clinical or sub-clinical symptoms of the state, disorder or condition developing in a mammal that may be afflicted with or predisposed to the state, disorder or condition but does not yet experience or display clinical or subclinical symptoms of the state, disorder or condition; or (2) inhibiting the state, disorder or condition, i.e., arresting, reducing or delaying the development of the disorder or a relapse thereof (in case of maintenance treatment) or at least one clinical or sub-clinical symptom thereof; or (3) relieving the disorder, i.e., causing regression of the state, disorder or condition or at least one of its clinical or sub-clinical symptoms. The benefit to a subject to be treated is either statistically significant or at least perceptible to the patient or to the physician.

As used herein "combination therapy" means the treatment of a subject in need of treatment with a certain composition or drug in which the subject is treated or given one or more other compositions or drugs for the disease in conjunction with the first and/or in conjunction with one or more other therapies, such as, e.g., an antidepressant agent. Such combination therapy can be sequential therapy wherein the patient is treated first with one treatment modality (e.g., drug or therapy), and then the other (e.g., drug or therapy), and so on, or all drugs and/or therapies can be administered simultaneously. In either case, these drugs and/or therapies are said to be "coadministered." It is to be understood that "coadministered" does not necessarily mean that the drugs and/or therapies are administered in a combined form (i.e., they may be administered separately or together to the same or different sites at the same or different times).

As used herein, the phrase "pharmaceutically acceptable" refers to molecular entities and compositions that are generally believed to be physiologically tolerable and do not typically produce an allergic or similar untoward reaction, such as gastric upset, dizziness and the like, when administered to a human. The term "pharmaceutically acceptable derivative" as used herein means any pharmaceutically acceptable salt, solvate or prodrug, e.g. ester, of a compound of the present disclosure, which upon administration to the recipient is capable of providing (directly or indirectly) a compound of the present disclosure, or an active metabolite or residue thereof. Such derivatives are recognizable to those skilled in the art, without undue experimentation. Derivatives are described, for example, in Burger's Medicinal Chemistry and Drug Discovery, 5th Edition, Vol 1: Principles and Practice, which is incorporated herein by reference to the extent of teaching such derivatives. Preferred pharmaceutically acceptable derivatives include salts, solvates, esters, carbamates, and phosphate esters. Particularly preferred pharmaceutically acceptable derivatives are salts, solvates, and esters. Most preferred pharmaceutically acceptable derivatives are salts and esters.

A "therapeutically effective amount" of a drug is an amount effective to demonstrate a desired activity of the drug. According to the instant disclosure, a therapeutically effective amount of a compound disclosed herein (e.g., NPY, or NPY analog, active fragment of NPY or NPY analog, or other NPY receptor ligand (including, e.g., Y1 receptor agonist and/or Y2 receptor antagonist)) is an amount effective to alleviate, i.e., noticeably reduce or eliminate one or more of the patient's symptoms associated with a mood and/or anxiety disorder. The therapeutically effective amount can vary depending on the compound, the disorder and its severity, and the age, weight, physical condition and responsiveness of the subject (e.g., patient) to be treated.

The term "about" or "approximately" means within an acceptable range for the particular value as determined by one of ordinary skill in the art, which will depend in part on how the value is measured or determined, e.g., the limitations of the measurement system. For example, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5 fold, and more preferably within 2 fold, of a value. Unless otherwise stated, the term 'about' means within an acceptable error range for the particular value.

The details of one or more embodiments of the present disclosure are set forth in the description and claims below.

### DETAILED DESCRIPTION

The following descriptions are provided for clarity and illustrative purposes only, and are not intended to limit the scope of the present disclosure.

### Overview

As described above, there is a need in the art for improved compositions and methods for the treatment of mood and anxiety disorders such as, e.g., Major Depressive Disorder, Panic Disorder, Specific phobia, Social Phobia, Obsessive-Compulsive Disorder (OCD), PTSD, acute stress disorder, and generalized anxiety disorder. Presently provided are compositions and formulations comprising high doses of neuropeptide Y (NPY) (or NPY analog, active fragment or conjugate of NPY or NPY analog, or other NPY receptor ligand (including, e.g., Y1 receptor agonist and/or Y2 receptor antagonist)) (e.g., greater than 0.1 mg/kg, greater than 0.5 mg/kg, greater than 1.0 mg/kg, or greater than 1.5 mg/kg). The presently disclosed compositions are preferably formulated such that they are suitable for intranasal administration. It is particularly preferred that the present compositions and formulations are administered to the olfactory region of the nasal cavity. It is also particularly preferred that entry of the compositions and formulations and/or the active ingredient (e.g., NPY (or NPY analog, active fragment of NPY or NPY analog, or other NPY receptor ligand (including, e.g., Y1 receptor agonist and/or Y2 receptor antagonist))) contained in the compositions and formulations disclosed herein into the systemic circulatory system is minimized. As discussed in detail below, delivery of the compositions and formulations to the olfactory region and the minimization of entry into the systemic circulatory system can be achieved by the specific route of administration and/or the device used to administer the compositions and formulations.

Many therapeutic drugs that are administered systemically (e.g., via intravenous injection) have difficulty reaching the central nervous system (CNS) from the systemic blood circulation, because the blood-brain barrier (BBB) and the blood-cerebrospinal fluid barrier (BCSFB) form a very effective barrier that prevents most molecules from passing through. Thus, efficient delivery of peptide drugs such as NPY to the brain in therapeutically effective amounts can be difficult to achieve when the peptide is administered systemically. This is particularly true for NPY, because NPY is a vasoconstrictor, and constriction of the blood vessels further reduces its transport into the brain. Moreover, while some of the NPY administered systemically (e.g., via intravenous route) may reach the brain, it can have side effects that are undesirable, including undesirable effects on the cardiovascular system.

In certain embodiments, while not intending to be bound by any one particular theory or mechanism of action, it is believed that the presently described compositions and formulations comprising NPY (or NPY analog, active fragment or conjugate of NPY or NPY analog, or other NPY receptor ligand (including, e.g., Y1 receptor agonist and/or Y2 receptor antagonist)) and methods of their administration (i.e., intranasal administration whereby drug is delivered to the olfactory region of the nasal cavity) are particularly effective because NPY (or NPY analog, active fragment or conjugate of NPY or NPY analog, or other NPY receptor ligand (including, e.g., Y1 receptor agonist and/or Y2 receptor antagonist)) can be rapidly and directly transported into the CNS in minutes via the unique connection of the olfactory region and the trigeminal nervous system, thereby bypassing the blood-brain barrier, and affecting multiple sites within the brain. The olfactory region in humans is situated in the roof of the nasal cavity. Although only 3% of the nasal cavity is occupied by olfactory epithelium, this route is direct, since the olfactory neurons do not have a synapse between the receptive element and the afferent path. The feasibility of using olfactory neurons to serve as a direct drug transport route to the CSF and brain has been investigated extensively during the last decades. It is now understood that the mechanisms of drug uptake into the brain from the nasal cavity mainly through two different pathways. One is the systemic pathway by which some of the drug is absorbed into the systemic circulation by the rich vasculature of the respiratory epithelium and subsequently reaches the brain by crossing the BBB. The other is the olfactory pathway by which the drug is directly delivered to brain tissue, bypassing the BBB. Drugs that move across olfactory epithelial cells may simply move slowly through tight interstitial space of cells, or across the cell membrane by endocytosis, or transported by vesicle carriers and neurons. It is particularly preferred that the compositions and formulations described herein be administered such that they reach the olfactory region, thereby facilitating direct delivery of the drug to the brain.

Intranasal delivery of peptide drugs (e.g., NPY) is also attractive because it permits non-invasive, rapid systemic absorption, fast onset of action, avoidance of first-pass metabolism, increased drug bioavailability, and less systemic side effects. *See,* Wen, M.M. "Olfactory Targeting Through Intranasal Delivery of Biopharmaceutical Drugs to the Brain - Current Development; Discov Med 11(61):497-503, June 2011).

### Neuropeptide Y (NPY)

NPY is one of the most abundant peptides in the central nervous system (CNS) of both rodents and humans. NPY interacts with noradrenalin, serotonin and dopamine and has neuromodulatory effect on multiple brain functions including feeding behavior, water consumption, learning and memory, locomotion, body temperature regulation, sexual behavior, emotional behavior, neuronal excitability, cardiovascular homeostasis, hormone secretion and circadian rhythms. In addition, it has been suggested that NPY plays a role in psychiatric disorders including depression, eating disorders, anxiety and epilepsy. NPY is abundantly expressed in the peripheral nervous system (PNS) in particular in nerves innervating small and medium sized blood vessles. NPY has been indicated to modulate neuronal excitability in many areas of the CNS, involving multiple pre- and post synaptic mechanisms. NPY modulates the release of different neurotransmitters such as glutamate, GABA, noradrenalin/adrenalin, dopamine, somatostatin, serotonin, nitric oxide, growth hormone and corticotropin releasing factor. NPY has been shown to play an important neuroprotective role e. g. in pathological conditions such as ischemia and epilepsy, but also in psychiatric disorders such as depression, anxiety, alcohol abuse - dependence and withdrawal.

Dysregulation of NPY in depression has been indicated by different study paradigms. NPY is involved in memory processing and affects the state of hedonia/anhedonia by changing dopamine concentration and release in striatum, both functions being affected during depression. Thus all antidepressant treatments so far tested in animal models show behavioral effects and, in parallel, increased NPY expression in selected brain regions.

NPY exerts its action by binding to NPY-receptors consisting in five Y-receptor subtypes (Y1, Y2, Y4, Y5 and y6) and one receptor subtype (Y3) that has not yet been cloned but pharmacologically characterized only in the nucleus of the solitary tract, all belonging to the superfamily of G-protein coupled receptors. NPY receptors are highly expressed in the CNS, including in limbic brain regions important for mood, motivation, arousal and fear - regions also highly implicated in mood and anxiety disorders. Key limbic brain regions expressing NPY receptors include the hippocampus, amygdala and hypothalamus. The Y1 and Y2 receptors are both located pre- and post-synaptically with a predominant postsynaptic location of the Y1 and a predominantly pre-synaptic location of the Y2 receptor.

NPY as a whole has the highest affinity to the Y1-receptor, whereas its C-terminal fragment NPY13-36 preferably binds to Y2-receptors. The peptide YY (PYY), which together with the pancreatic polypeptide (PP) forms the family of pancreatic peptides, binds preferentially to Y5 receptors, whereas the Y4 receptor shows selectivity towards PP. The y6 receptor (y6R) protein is truncated in most mammals, and it is functional only in the mouse and rabbit. The Y1 receptor is most abundantly expressed in the mammalian CNS and is the NPY receptor most implicated in mood and anxiety disorders.

NPY receptors exert their effect via different second messenger systems. For the Y1 receptor the most common actions are inhibition of adenylate cyclase, via the pertussis toxin sensitive GTP binding protein Gi/Go and mobilization of Ca 2++ from intracellular stores. In addition, Y1 receptors stimulate the mitogen-activated protein kinase (MAPK) pathways by inducing phosphorylation of extracellularly regulated kinase (ERK), an effect that has been shown to be dependent on PI-3-kinase.

In humans, NPY is a 36-amino acid polypeptide, rich in tyrosine residues, with a molecular weight of 4271.74 Daltons having the sequence: YPSKPDNPGEDAPAEDMARYYSALRHYINLITRQRY (SEQ ID NO: 1) (GenBank® Accession No. 1006203A). While polymorphisms of NPY and its receptors have been identified, they are some of the most highly conserved molecules known and thus chemically very similar across species. Thus, the present disclosure contemplates compositions for treating a mood and/or anxiety disorder (e.g., Major Depressive Disorder, Panic Disorder, Specific phobia, Social Phobia, OCD, PTSD, acute stress disorder, and generalized anxiety disorder) that comprise any mammalian NPY. Mammalian NPY can be synthetic or naturally derived (e.g., isolated from a mammal or mammals). In a preferred embodiment, a composition or formulation disclosed herein comprises synthetic human NPY. An exemplary commercial source of mammalian NPY (including human NPY) is Bachem (Torrance, CA).

Also contemplated for use in the compositions, formulations and methods disclosed herein are analogs of NPY. Examples of analogs of NPY are those that have at least one amino acid addition, insertion, substitution or deletion in the amino acid sequence of NPY. In other embodiments, an analog of NPY has at least 2, at least 3, at least 4, at least 5, at least 6, at least 7, at least 8, at least 9, at least 10, or more amino acid substitutions. An analog of NPY is contemplated for use herein if the analog maintains the ability to activate signal transduction through the Y1 receptor (i.e., to activate the Y1 receptor). In some embodiments, an amino acid substitution in NPY will be a conservative substitution. A conservative substitution is the substitution of one amino acid for another with similar characteristics. Conservative substitutions include substitutions within the following groups: valine, alanine and glycine; leucine, valine, and isoleucine; aspartic acid and glutamic acid; asparagine and glutamine; serine, cysteine, and threonine; lysine and arginine; and phenylalanine and tyrosine. The non-polar hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Any substitution of one member of the above-mentioned polar, basic or acidic groups by another member of the same group can be deemed a conservative substitution. By contrast, a non-conservative substitution is a substitution of one amino acid for another with dissimilar characteristics.

Also encompassed herein are polypeptides that have at least 85% sequence identity to NPY (e.g., to SEQ ID NO: 1), e.g., a peptide or peptide fragment having at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or greater sequence identity to SEQ ID NO: 1.

As used herein, "sequence identity" or "identity" in the context of two nucleic acid or polypeptide sequences makes reference to a specified percentage of residues in the two sequences that are identical when aligned for maximum correspondence over a specified comparison window, as measured by sequence comparison algorithms or by visual inspection. When percentage of sequence identity is used in reference to polypeptides it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity." Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, California).

As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

In certain embodiments, a fragment of a polypeptide disclosed herein has "substantial identity" with a reference sequence (e.g., NPY, e.g., SEQ ID NO: 1). The term "substantial identity" in the context of a peptide indicates that a peptide comprises a sequence with at least 70%, 71%, 72%, 73%, 74%, 75%, 76%, 77%, 78%, or 79%, preferably 80%, 81%, 82%, 83%, 84%, 85%, 86%, 87%, 88%, or 89%, more preferably at least 90%, 91%, 92%, 93%, or 94%, or even more preferably, 95%, 96%, 97%, 98% or 99%, sequence identity to the reference sequence over a specified comparison window. Preferably, optimal alignment is conducted using the homology alignment algorithm of Needleman and Wunsch, J. Mol. Biol. 48:443 (1970). An indication that two peptide sequences are substantially identical is that one peptide is immunologically reactive with antibodies raised against the second peptide. Thus, a peptide is substantially identical to a second peptide, for example, where the two peptides differ only by a conservative substitution. One of skill in the art will recognize that these values can be appropriately adjusted to determine corresponding identity of proteins encoded by two nucleotide sequences by taking into account codon degeneracy, amino acid similarity, reading frame positioning, and the like. Substantial identity of amino acid sequences for these purposes normally means sequence identity of at least 70%, more preferably at least 80%, 90%, and most preferably at least 95%.

Methods for alignment of sequences for comparison are well known in the art. For example, the determination of percent identity between any two sequences can be accomplished using a mathematical algorithm. Non-limiting examples of such mathematical algorithms are the algorithm of Myers and Miller, CABIOS, 4:11 (1988); the local homology algorithm of Smith et al., Adv. Appl. Math., 2:482 (1981); the homology alignment algorithm of Needleman and Wunsch, JMB, 48:443 (1970); the search-for-similarity-method of Pearson and Lipman, Proc. Natl. Acad. Sci. USA, 85:2444 (1988); the algorithm of Karlin and Altschul, Proc. Natl. Acad. Sci. USA, 87:2264 (1990), modified as in Karlin and Altschul, Proc. Natl. Acad. Sci. USA, 90:5873 (1993).

Computer implementations of these mathematical algorithms can be utilized for comparison of sequences to determine sequence identity. Such implementations include, but are not limited to: CLUSTAL in the PC/Gene program (available from Intelligenetics, Mountain View, California); the ALIGN program (Version 2.0) and GAP, BESTFIT, BLAST, FASTA, and TFASTA in the Wisconsin Genetics Software Package, Version 8 (available from Genetics Computer Group (GCG), 575 Science Drive, Madison, Wisconsin, USA). Alignments using these programs can be performed using the default parameters. The CLUSTAL program is well described by Higgins et al., Gene, 73:237 (1988); Higgins et al., CABIOS, 5:151 (1989); Corpet et al., Nucl. Acids Res., 16:10881 (1988); Huang et al., CABIOS, 8:155 (1992); and Pearson et al., Meth. Mol. Biol., 24:307 (1994). The ALIGN program is based on the algorithm of Myers and Miller, supra. The BLAST programs of Altschul et al., JMB, 215:403 (1990); Nucl. Acids Res., 25:3389 (1990), are based on the algorithm of Karlin and Altschul supra.

Software for performing BLAST analyses is publicly available through the National Center for Biotechnology Information (on the WorldWideWeb at ncbi.nlm.nih.gov). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold. These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when the cumulative alignment score falls off by the quantity X from its maximum achieved value, the cumulative score goes to zero or below due to the accumulation of one or more negative-scoring residue alignments, or the end of either sequence is reached.

In addition to calculating percent sequence identity, the BLAST algorithm also performs a statistical analysis of the similarity between two sequences. One measure of similarity provided by the BLAST algorithm is the smallest sum probability (P(N)), which provides an indication of the probability by which a match between two nucleotide or amino acid sequences would occur by chance. For example, a test nucleic acid sequence is considered similar to a reference sequence if the smallest sum probability in a comparison of the test nucleic acid sequence to the reference nucleic acid sequence is less than about 0.1, more preferably less than about 0.01, and most preferably less than about 0.001.

To obtain gapped alignments for comparison purposes, Gapped BLAST (in BLAST 2.0) can be utilized as described in Altschul et al., Nucleic Acids Res. 25:3389 (1997). Alternatively, PSI-BLAST (in BLAST 2.0) can be used to perform an iterated search that detects distant relationships between molecules. See Altschul et al., supra. When utilizing BLAST, Gapped BLAST, PSI-BLAST, the default parameters of the respective programs (e.g. BLASTN for nucleotide sequences, BLASTX for proteins) can be used. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix. See the WorldWideWeb at ncbi.nlm.nih.gov. Alignment may also be performed manually by inspection.

Exemplary analogs of NPY that are known to be Y1 receptor agonists, and can be potentially used to treat a mood and/or anxiety disorder (e.g., Major Depressive Disorder, Panic Disorder, Specific phobia, Social Phobia, OCD, PTSD, acute stress disorder, and generalized anxiety disorder) are disclosed in Table 1, below:

**Table 1: Analogs of NPY**

| **Bachem (Torran ce, CA) Catalog No.** | **Analog Name** | **Related mammalian sequence(s)** | **Structure** | **Specificity** | **Related Publications** |
|---|---|---|---|---|---|
| H-3306 | (Leu³¹,Pro³⁴)-Neuropeptide Y | Human Rat | H-Tyr-Pro-Ser-Lys-Pro-Asp-Asn-Pro-Gly-Glu-Asp-Ala-Pro-Ala-Glu-Asp-Met-Ala-Arg-Tyr-Tyr-Ser-Ala-Leu-Arg-His-Tyr-Ile-Asn-Leu-Leu-Thr-Arg-Pro-Arg-Tyr-NH₂ | Specific Y₁ receptor agonist. | J.Fuhlendorff et al., Proc. Natl. Acad. Sci. USA, 87, 182 (1990) |
| | | | | | M.Labelle et al., Peptides, 18, 801 (1997) |
| | | | | | J.B.Buckwalt |
| | | | (SEQ ID NO: 2) Trifluoroacetate salt | | er et al., Am. J. Physiol. Heart Circ. Physiol., 287, H144 (2004) |
| H-8575 | (Lcu³¹,Pro³⁴)-Neuropeptide Y | Porcine | H-Tyr-Pro-Scr-Lys-Pro-Asp-Asn-Pro-Gly-Glu-Asp-Ala-Pro-Ala-Glu-Asp-Leu-Ala-Arg-Tyr-Tyr-Ser-Ala-Leu-Arg-His-Tyr-Ile-Asn-Leu-Leu-Thr-Arg-Pro-Arg-Tyr-NH2 (SEQ ID NO: 3) Trifluoroacetate salt | This NPY analog is a highly potent and selective ligand for Y₁ receptors in different cell lines and tissues. It has therefore been used to detect the NPY/PYY receptor subtype distribution, e.g., in rat brain. The peptide is also active in vivo, showing an even higher potency than NPY in increasing blood pressure in anesthetized rats. | J.Fuhlendorff et al., Proc. Natl. Acad. Sci. USA, 87, 182 (1990) |
| | | | | | D.R.Gehlert et al., Neurochem. Int., 21, 45 (1992) |
| H-8580 | (Pro³⁴)-Neuropeptide Y | Porcine | H-Tyr-Pro-Ser-Lys-Pro-Asp-Asn-Pro-Gly-Glu-Asp-Ala-Pro-Ala-Glu-Asp-Leu-Ala-Arg-Tyr-Tyr-Ser-Ala-Leu-Arg-His-Tyr-Ile-Asn-Leu-Ile-Thr-Arg-Pro-Arg-Tyr-NH₂ (SEQ ID NO: 4) Trifluoroacetate salt | (Pro³⁴)-NPY is a selective NPY agonist at Y₁ or postjunctional receptors, as seen in a simultaneous study of post-and prejunctional actions in anesthetized rats and dogs. Like NPY, the analog increased blood pressure, but, in contrast, showed no inhibitory effects on cardiac vagal action. | E.K.Potter et al., Eur. J. Pharmacol., 1 93, 15 (1991) |
| H-3972 | Tyr-Lys-Gly-(Cyclo(Glu²⁶-Lys²⁹),Pro³⁴)-Neuropeptide Y (25-36) | | H-Tyr-Lys-Gly-Arg-cyclo(-Glu-Tyr-Ilc-Lys)-Leu-Ile-Thr-Arg-Pro-Arg-Tyr-NH₂ (SEQ ID NO: 5) Trifluoroacetate salt | This truncated and cyclized NPY analog specifically bound to Y₁ receptors with a Ki of 16 nM. As an NPY agonist it inhibited norepinephrine-stimulated cAMP accumulation in SK-N-MC cells with an EC₅₀ of 12 nM. | D.A.Kirby et al., J. Med. Chem., 40, 210 (1997) |

Particularly preferred analogs that are Y1 receptor agonists for use as described herein include but are not limited to [Leu31,Pro34]-Neuropeptide Y (see, J.Fuhlendorff et al., *supra*), [Pro30, Nle31, Bpa32, Leu34]NPY(28-36), [Pro30, Nal32, Leu34]NPY(28-36), [Pro30, Nle31, Nal32, Leu34]NPY(28-36) (see, e.g., Zwanziger, D. et al. "First selective agonist of the neuropeptide Y1-receptor with reduced size" J. Pept. Sci. 2009; 15: 856-866).

An exemplary Y2 receptor antagonist is described in Brothers SP, Wahlestedt C. EMBO Mol Med. 2010 Nov;2(11):429-39. The synthesis of several potent and selective small molecule NPY Y2 receptor antagonists has also been previously reported by Mittapalli et al. (Bioorg Med Chem Lett. 2012 Jun 15;22(12):3916-20); the authors described highly potent and selective small molecule Y2 receptor antagonists, including 16 (CYM 9484) and 54 (CYM 9552) with IC₅₀ values of 19 nM and 12 nM, respectively. These and other antagonists of the Y2 receptor are contemplated for use in the methods disclosed herein.

Also encompassed by the present disclosure are fragments of NPY and fragments of NPY analogs (e.g., those described above). A fragment of NPY or of a NPY analog disclosed herein has an amino acid sequence, the length of which is fewer than the full length NPY sequence, and maintains the ability to bind and activate the Y1 receptor. Thus, for example, a fragment of human NPY has a length of less than 36 amino acids, e.g., about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, or about 35 amino acids. Particularly preferred are N-terminal fragments of NPY consisting of at least about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, or about 35 amino acids.

The present disclosure also encompasses conjugates of NPY and other modified forms of NPY or NPY analogs that are agonists of the Y1 receptor. For example, NPY, NPY analog, or fragment thereof, or other NPY receptor ligand (including, e.g., Y1 receptor agonist and/or Y2 receptor antagonist) can be administered as a conjugate with a mucoadhesive polymer or chitosan. These and other formulations of NPY are discussed in detail, below.

Also contemplated for use herein are small molecule ligands of the NYP receptors (e.g., Y1 agonists and Y2 antagonists). "Small molecules" are a class of chemical agents or compounds that are typically organic, non-peptide molecules, having a molecular weight less than 10,000 Da, preferably less than 5,000 Da, more preferably less than 1,000 Da, and most preferably less than 500 Da. This class of modulators includes chemically synthesized molecules, for instance, compounds from combinatorial chemical libraries. Synthetic compounds may be rationally designed or identified utilizing the screening methods described below. Alternative appropriate modulators of this class are natural products, particularly secondary metabolites from organisms such as plants or fungi, which can also be identified by screening compound libraries for binding to and/or interacting with one more NPY receptors (e.g., as agonists or antagonists), as described below. Methods for generating and obtaining small molecules are well known in the art (Schreiber, Science 2000; 151:1964-1969; Radmann et al., Science 2000; 151:1947-1948). Small molecules that can act as NPY receptor ligands (including, e.g., Y1 receptor agonist and/or Y2 receptor antagonists) can be further optimized using classical ligand-based design approaches (such as, e.g., virtual screening, pharmacophore modeling, quantitative structure-activity relationship (QSAR), etc.) as well as by synthesizing combinatorial libraries (for review see, e.g., Klabunde et al., Chembiochem. 2002; 3:928-44).

The above-described examples of NPY, NPY analogs, active fragments or conjugates of NPY or NPY analogs, or other NPY receptor ligands (including, e.g., Y1 receptor agonist and/or Y2 receptor antagonist) encompassed by the present disclosure are for use in a method for treating a mood and/or anxiety disorder (e.g., Major Depressive Disorder, Panic Disorder, Specific phobia, Social Phobia, OCD, PTSD, acute stress disorder, and generalized anxiety disorder). The skilled artisan will understand how to design and test additional Y1 agonists. Y1 agonists can be peptide-based agonists and/or small molecule agonists. Methods for testing the functional activity of an agonist (i.e., screening assays) are known in the art and discussed below.

Screening assays for testing the activity of NPY, NPY analogs, conjugates and fragments thereof, and other NPY receptor ligands disclosed herein are known in the art. See, e.g., the publication by Abounader et al. (British Journal of Pharmacology (1995) 116, 2245 - 2250), which describes in detail an *in vitro* functional assay for testing the functional activity of NPY and NPY analogs for ability to activate (or antagonize) the Y1 receptor. This assay can be used to test the functional activity of NPY (or NPY analog, active fragment or conjugates of NPY or NPY analog, or other Y1 receptor agonist). Similarly, assays for Y2 ligands have been previously described in Brothers *et al.* (*supra*) and Mittapalli *et al.* (*supra*).

NPY, NPY analogs, active fragments of NPY or NPY analogs, or other NPY receptor ligands (including, e.g., Y1 receptor agonist and/or Y2 receptor antagonist) disclosed herein, can be modified in any suitable way known in the art, e.g., to enhance bioavailability and/or ability to enter the brain via the olfactory epithelium and/or the trigeminal nervous system and/or to minimize entry into the circulatory system.

### Formulations

While it is possible to use a composition disclosed herein (e.g., a composition comprising NPY (or NPY analog, active fragment or conjugate of NPY or NPY analog, or other Y1 receptor agonist) (or NPY analog, active fragment or conjugate of NPY or NPY analog, or other NPY receptor ligand (including, e.g., Y1 receptor agonist and/or Y2 receptor antagonist)) for therapy as is, it may be preferable to formulate the composition in a pharmaceutical formulation, e.g., in admixture with a suitable pharmaceutical excipient, diluent, or carrier selected with regard to the intended route of administration and standard pharmaceutical practice. Suitable carriers and their formulations are described in Remington: The Science and Practice of Pharmacy, 21st ed., 2005, Lippincott, Williams & Wilkins, Phila., PA.

For *in vivo* administration to humans, the compositions can be formulated according to known methods used to prepare pharmaceutically useful compositions. Compositions may be designed to be short-acting, fast-releasing, long-acting, or sustained-releasing. Thus, pharmaceutical formulations may also be formulated for controlled release or for slow release.

In a preferred embodiment, NPY (or NPY analog, active fragment or conjugate of NPY or NPY analog, or other NPY receptor ligand (including, e.g., Y1 receptor agonist and/or Y2 receptor antagonist)) is formulated for intranasal administration (i.e., is formulated to be suitable for intranasal administration). In a specific embodiment, the present disclosure provides a pharmaceutical formulation comprising: (a) at least 0.005 mg/kg NPY and (b), a pharmaceutically acceptable carrier, wherein the pharmaceutical formulation is suitable for intranasal administration. In another embodiment, the pharmaceutical formulation comprises (a) at least 0.05 mg/kg NPY and (b), a pharmaceutically acceptable carrier, wherein the pharmaceutical formulation is suitable for intranasal administration. In another embodiment, the pharmaceutical formulation comprises (a) at least 0.01 mg/kg NPY and (b), a pharmaceutically acceptable carrier, wherein the pharmaceutical formulation is suitable for intranasal administration. In another embodiment, the pharmaceutical formulation comprises (a) at least 0.5 mg/kg NPY and (b), a pharmaceutically acceptable carrier, wherein the pharmaceutical formulation is suitable for intranasal administration. In another embodiment, the pharmaceutical formulation comprises (a) at least 0.1 mg/kg NPY and (b), a pharmaceutically acceptable carrier, wherein the pharmaceutical formulation is suitable for intranasal administration. In another embodiment, the pharmaceutical formulation comprises (a) 1 mg/kg NPY and (b), a pharmaceutically acceptable carrier, wherein the pharmaceutical formulation is suitable for intranasal administration. Suitable formulations for intranasal administration are discussed in detail, *infra.* In a further embodiment, NPY can be formulated with a mucosal penetration enhancer to facilitate delivery of the drug (or drugs, when more than one active ingredient is contained in the formulation). The formulation can also be prepared with pH optimized for solubility, drug stability, absorption through nasal mucosa, and other considerations.

In other embodiments, provided herein is an intranasal dosage forms containing NPY and a pharmaceutically acceptable diluent. For example, the dosage forms can include NPY in a dosage range from about 0.005 milligrams per kilogram of body weight (mg/kg) to about 2 mg/kg, from about 0.005 mg/kg to about 1.5 mg/kg, from about 0.005 mg/kg to about 1 mg/kg, from about 0.01 mg/kg to about 1.0 mg/kg, from about 0.05 mg/kg to about 1.0 mg/kg, or from about 0.05 mg/kg to about 0.5 mg/kg. In another embodiment, the dosage forms can include NPY in a dosage range from about 0.05 mg to about 200 mg, from about 0.05 mg to about 150 mg, from about 0.05 mg to about 125 mg, from about 0.5 mg to about 100 mg, from about 1 mg to about 100 mg, from about 1 mg to about 50 mg, from about 1 mg to about 40 mg, from about 1 mg to about 35 mg, from about 1 mg to about 30 mg, from about 1 mg to about 25 mg, from about 1 mg to about 20 mg, from about 1 mg to about 15 mg, from about 1 mg to about 10 mg, from about from about 1 mg to about 5 mg, from about 2 mg to about 35 mg, from about 3 mg to about 30 mg, from about 4 mg to about 25 mg, from about 5 mg to about 20 mg, from about 6 mg to about 15 mg, from about 7 mg to about 10 mg, from about 8 mg to about 20 mg, from about 9 mg to about 20 mg, or from about 10 mg to about 20 mg.

Accordingly, in one aspect, a pharmaceutical composition or formulation disclosed herein comprises at least one active composition disclosed herein (e.g., a composition comprising NPY (or NPY analog, active fragment or conjugate of NPY or NPY analog, or other NPY receptor ligand (including, e.g., Y1 receptor agonist and/or Y2 receptor antagonist)) in association with a pharmaceutically acceptable excipient, diluent, and/or carrier. The excipient, diluent and/or carrier must be "acceptable" in the sense of being compatible with the other ingredients of the formulation and not deleterious to the recipient thereof.

In certain embodiments, the present disclosure provides liquid or powder aerosol formulations and dosage forms for intranasal administration (e.g., for use in treating subjects suffering from a mood and/or anxiety disorder). In general such dosage forms NPY (or NPY analog, active fragment or conjugate of NPY or NPY analog, or other NPY receptor ligand (including, e.g., Y1 receptor agonist and/or Y2 receptor antagonist)) in a pharmaceutically acceptable diluent. Pharmaceutically acceptable diluents in such liquid aerosol formulations include but are not limited to sterile water, saline, buffered saline, dextrose solution, and the like. In a specific embodiment, a diluent that may be used in the present disclosure and/or in a pharmaceutical formulation of the present disclosure is phosphate buffered saline or a buffered saline solution generally between the pH 7.0-8.0 range, or water. The present disclosure contemplates the use of any suitable diluent known in the art for intranasal administration.

The formulations of the present disclosure may also include other agents, ingredients, and/or components, e.g., that are useful for pH maintenance, solution stabilization, or for the regulation of osmotic pressure, including, but not limited to salts, such as sodium chloride, or potassium chloride, and carbohydrates, such as glucose, galactose or mannose, and the like.

In other embodiments, the aerosol formulation of NPY (or NPY analog, active fragment or conjugate of NPY or NPY analog, or other NPY receptor ligand (including, e.g., Y1 receptor agonist and/or Y2 receptor antagonist)) is provided as a dry powder aerosol formulation in which the NPY (or NPY analog, active fragment or conjugate of NPY or NPY analog, or other NPY receptor ligand (including, e.g., Y1 receptor agonist and/or Y2 receptor antagonist)) is present as a finely divided powder. The dry powder formulation can further comprise a bulking agent, such as, but not limited to, lactose, sorbitol, sucrose and mannitol.

The aerosolization of a liquid or a dry powder formulation can include a propellant. The propellant may be any propellant generally used in the art. Specific non-limiting examples of such useful propellants are a chlorofluorocarbon, a hydrofluorocarbon, a hydochlorofluorocarbon, or a hydrocarbon, including trifluoromethane, dichlorodiflouromethane, dichlorotetrafluoroethanol, and 1,1,1,2-tetraflouroethane, or combinations thereof.

Systems of aerosol delivery, such as a pressurized metered dose inhaler and a dry powder inhaler are disclosed in Newman, S. P., Aerosols and the Lung, Clarke, S. W. and Davia, D. editors, pp. 197-22 and can be used in connection with the present disclosure.

In certain embodiments, NPY (or NPY analog, active fragment or conjugate of NPY or NPY analog, or other NPY receptor ligand (including, e.g., Y1 receptor agonist and/or Y2 receptor antagonist)) may be formulated with a "mucosal penetration enhancer," i.e., a reagent that increases the rate or facility of transmucosal penetration of NPY (or NPY analog, active fragment or conjugate of NPY or NPY analog, or other NPY receptor ligand (including, e.g., Y1 receptor agonist and/or Y2 receptor antagonist)), such as but not limited to, a bile salt, fatty acid, surfactant or alcohol. In specific embodiments, the permeation enhancer can be sodium cholate, sodium dodecyl sulphate, sodium deoxycholate, taurodeoxycholate, sodium glycocholate, dimethylsulfoxide or ethanol. Another example includes, e.g., N-tridecyl-beta-D-maltoside.

In another embodiment NPY (or NPY analog, active fragment or conjugate of NPY or NPY analog, or other NPY receptor ligand (including, e.g., Y1 receptor agonist and/or Y2 receptor antagonist)) can be formulated with Solutol HS 15, which is a nonionic solubilizer and emulsifying agent obtained by reacting 15 moles of ethylene oxide with 1 mole of 12-hydroxy stearic acid.

In certain embodiments, NPY (or NPY analog, active fragment or conjugate of NPY or NPY analog, or other NPY receptor ligand (including, e.g., Y1 receptor agonist and/or Y2 receptor antagonist)) may be formulated with a mucoadhesive polymer such as but not limited to polyacrylic acid, Carbopol, Hyaluronan and carboxymethylcellulose. Mucoadhesive polymers can increase the mucosal contact time and prolong the residence time of the dosage forms in the nasal cavity.

Another example of a mucoadhesive polymer is chitosan. Chitosan is a nontoxic and has absorption enhancing and mucoadhesive properties. Chitosan hydrochloride in combination with hydroxypropyl beta-cyclodextrin or Chitosan in combination with hydroxylpropylmethyl cellulose can also be used. In another embodiment, chitosan glutamate UP G213 (NovaMatrix, Norway) can be used to prepare a chitosan-NPY formulation. Chitosan stock solution (10 mg/ml) can be prepared by adding 3 ml of 0.9% NaCl solution into a vial containing 30 mg of chitosan glutamate 0213 and magnetically stirred until the chitosan is dissolved. Lyophilized Neuropeptide Y (250 µg) is then reconstituted in 0.125 ml of 0.9% NaCI solution and transferred into an HPLC sample vial. Into the Neuropeptide Y solution, 0.125 ml of the chitosan stock solution is added. The final formulation contains 1 mg/ml Neuropeptide Y and 5 mg/ml chitosan glutamate 0213. The formulation can be administered, e.g., using a simple nasal spray or a nasal drop system, to a subject sitting with their head tilted back.

In another embodiment, NPY (or NPY analog, active fragment or conjugate of NPY or NPY analog, or other NPY receptor ligand (including, e.g., Y1 receptor agonist and/or Y2 receptor antagonist)) can be mixed with microcrystalline cellulose, freeze dried, and administered as a powder using a nasal powder spray (e.g., mono-poudre from Valois) and administered to a subject in a sitting position. The formulation may also be administered to a subject lying on his or her back with the head tilted back. In another embodiment, NPY (or an analog of NPY, NPY derivative, and/or NPY receptor ligand (including, e.g., Y1 receptor agonist and/or Y2 receptor antagonist)) is dissolved in a solution of methylcellulose (3%) in phosphate buffer (PH 6.5) which creates a bioadhesive gel system. This formulation can be administered with the nasal drop system with the subjects lying on their backs with the head tilted back. In another embodiment, NPY (or NPY analog, active fragment or conjugate of NPY or NPY analog, or other NPY receptor ligand (including, e.g., Y1 receptor agonist and/or Y2 receptor antagonist)) is dissolved in a dispersion of Sephadex 0-25 dextran microspheres. The dispersion is freeze dried and the formulation administered as a powder as above with the subject in sitting position, with the head tilted back. The microspheres are fractioned and the sieve fraction below 150 µm selected. The Sephadex microspheres are bioadhesive and have been shown to enhance absorption. Other considerations, such as construction of the delivery device, discussed *infra,* additional components in the formulation, and particle characteristics are also important. These aspects of intranasal administration of a drug are well known in the art, and manipulation of formulations, aerosolization means and construction of a delivery device require at most routine experimentation by one of ordinary skill in the art.

In another embodiment, NPY (or NPY analog, active fragment or conjugate of NPY or NPY analog, or other NPY receptor ligand (including, e.g., Y1 receptor agonist and/or Y2 receptor antagonist)) may be formulated with a vasoconstrictor, e.g., phenylephrine hydrochloride. It has been described that phenylephrine hydrochloride, a short-acting vasoconstrictor, showed remarkably reduced blood concentrations and increased CNS concentrations of hypocretin-1, a peptide involved in appetite and sleep regulation, and dipeptide L-Tyr-D-Arg, a morphine-like analgesic. In this case, the vasoconstrictor was used to enhance intranasal drug targeting to the CNS by limiting absorption into the systemic circulation and increasing the amount of neuropeptide available for direct transport into the CNS along olfactory pathways. In certain embodiment, a vasoconstrictor may be coadministered to a subject suffering from a mood and/or anxiety disorder (e.g., Major Depressive Disorder, Panic Disorder, Specific phobia, Social Phobia, OCD, PTSD, acute stress disorder, and generalized anxiety disorder).

In certain aspects, NPY (or NPY analog, active fragment or conjugate of NPY or NPY analog, or other NPY receptor ligand (including, e.g., Y1 receptor agonist and/or Y2 receptor antagonist)) is formulated with a dispersant. Preferably, the dispersant is pharmaceutically acceptable. Suitable dispersing agents are well known in the art, and include but are not limited to surfactants and the like. Such surfactants are generally used in the art to reduce surface induce aggregation of NPY caused by atomization of the solution forming the liquid aerosol and may be used in the methods and devices of the present disclosure. Examples of such surfactants include, but are not limited to, surfactants such as polyoxyethylene fatty acid esters and alcohols, and polyoxyethylene sorbitan fatty acid esters. Amounts of surfactants used will vary, being generally within the range or 0.001 and 4% by weight of the formulation. Suitable surfactants are well known in the art, and can be selected on the basis of desired properties, depending on the specific formulation, concentration of NPY, diluent (in a liquid formulation).

Formulations for use in the methods described herein can include other therapeutically or pharmacologically active ingredients in addition to NPY, such as but not limited to agents used for antidepressant therapy. Such second agents include, but are not limited to, antidepressants: e.g., biogenic amine non-selective reuptake inhibitors, e.g., tricyclic antidepressants like imipramine; serotonin selective reuptake inhibitors like fluoxetine (Prozac); diazepam; monoamine oxidase (MAO) inhibitors, e.g., iproniazid, clorgyline, phenelzine, tranylcypromine, and isocarboxazid; biogenic amine uptake blockers, e.g., tricyclic antidepressants such as desipramine, imipramine and amitriptyline; atypical antidepressants such as mirtazapine, nefazodone, bupropion; serotonin reuptake inhibitors e.g., fluoxetine, venlafaxine, and duloxetine; antipsychotic drugs such as phenothiazine derivatives (e.g., chlorpromazine (thorazine) and trifluopromazine)), butyrophenones (e.g., haloperidol (Haldol)), thioxanthene derivatives (e.g., chlorprothixene), S and dibenzodiazepines (e.g., clozapine); benzodiazepines; dopaminergic agonists and antagonists e.g., L-DOPA, cocaine, amphetamine, a-methyl-tyrosine, reserpine, tetrabenazine, benztropine, pargyline; noradrenergic agonists and antagonists e.g., clonidine, phenoxybenzamine, phentolamine, tropolone. NPY may also be formulated with benzodiazepine, lithium, lithium salts, carbamazepine, valproic acid, T3, or T4.

In another embodiment of the treatment methods, the compositions administered further comprise compounds, in particular drugs, reported to ameliorate or exacerbate the symptoms of oxidative stress disorder. Such compounds include reduced IS glutathione (GSH), glutathione precursors, e.g., N-acetylcysteine; antioxidants, e.g., vitamins E and C, beta carotene and quinones; inhibitors of lipid membrane peroxidation, e.g., 21-aminosteroid U74006F (tirilazad mesylate), and lazaroids; antioxidants such as mazindol; 2c dizocilpine maleate; selegiline; sulfhydryls N-acetyleysteine and cysteamine; dimethylthiourea; EUK-8 a synthetic, low molecular salen-manganese complex; synthetic manganese-based metalloprotein superoxide dismutase mimic, SC52608; free radical scavengers or suppressors, e.g., pegorgotein, tocotrienol, tocopheral, MDL 74,18, LY231617, MCI-186, AVS (nicaraven), allopurinol, rifampicin, oxypurinol, hypochlorous acid or recombinant human Cu, Zn-SOD.

While intranasal delivery is the preferred route of administration for the peptide agonists of the Y1 receptor (e.g., NPY, NPY analogs, fragments and conjugates thereof), other routes of administration are also contemplated, in particular for small molecules agonists of the Y1 receptor. For example, small molecules may be administered orally (although other routes of administration, e.g., intravenous) are also contemplated. For oral administration, compositions containing small molecules agonist of the Y1 receptor may take the form of tablets or capsules prepared by conventional means with pharmaceutically acceptable excipients such as binding agents (e.g., pregelatinized maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the compositions may take the form of tablets or lozenges formulated in conventional manner. The pharmaceutical compositions may be added to a retained physiological fluid such as blood or synovial fluid. In another embodiment, the active ingredient can be delivered in a vesicle, in particular a liposome (see Langer, Science 249:1527-1533 (1990); Treat et al., in Liposomes in the Therapy of Infectious Disease and Cancer, Lopez-Berestein and Fidler (eds.), Liss: New York, pp. 353-365 (1989); Lopez-Berestein, ibid., pp. 317-327; see generally ibid.).

### Dosages

Effective amounts of NPY (or NPY analog, active fragment or conjugate of NPY or NPY analog, or other NPY receptor ligand (including, e.g., Y1 receptor agonist and/or Y2 receptor antagonist)) in compositions including pharmaceutical formulations, include doses that partially or completely achieve the desired therapeutic, prophylactic and/or biological effect. In a specific embodiment, an effective amount of NPY (or NPY analog, active fragment or conjugate of NPY or NPY analog, or other NPY receptor ligand (including, e.g., Y1 receptor agonist and/or Y2 receptor antagonist)) administered to a subject with a mood and/or anxiety disorder (e.g., Major Depressive Disorder, Panic Disorder, Specific phobia, Social Phobia, OCD, PTSD, acute stress disorder, and generalized anxiety disorder) is effective for treating one or more signs or symptoms of the mood and/or anxiety disorder. The actual amount effective for a particular application depends on the condition being treated and the route of administration.

In certain aspect, the present disclosure provides for administration of a therapeutically effective dose of NPY (or NPY analog, active fragment or conjugate of NPY or NPY analog, or other NPY receptor ligand (including, e.g., Y1 receptor agonist and/or Y2 receptor antagonist)), i.e., a dose effective to treat a mood and/or anxiety disorder (e.g., Major Depressive Disorder, Panic Disorder, Specific phobia, Social Phobia, OCD, PTSD, acute stress disorder, and generalized anxiety disorder). Specific dosages may be adjusted depending on conditions of disease, i.e., the severity of the mood and/or anxiety disorder, the age, body weight, general health conditions, sex, and diet of the subject, dose intervals, administration routes, excretion rate, and combinations of drugs. Any of the dosage forms described herein containing effective amounts of NPY (or NPY analog, active fragment or conjugate of NPY or NPY analog, or other NPY receptor ligand (including, e.g., Y1 receptor agonist and/or Y2 receptor antagonist)), either alone or in combination with one or more active agents, are well within the bounds of routine experimentation and therefore, well within the scope of the instant invention.

An initial dose may be larger, followed by smaller maintenance doses. The dose may be administered as infrequently as weekly or biweekly, or fractionated into smaller doses and administered daily, several times daily, semi-weekly, bi-weekly, quarterly, etc., to maintain an effective dosage level. Preliminary doses can be determined according to animal tests, and the scaling of dosages for human administration can be performed according to art-accepted practices. In certain embodiments, a subject may be administered 1 dose, 2 doses, 3 doses, 4 doses, 5 doses, 6 doses or more of a NPY-containing composition described herein. However, other ranges are possible, depending on the subject's response to the treatment Moreover, an initial dose may be the same as, or lower or higher than subsequently administered doses of NPY of NPY (or NPY analog, active fragment or conjugate of NPY or NPY analog, or other NPY receptor ligand (including, e.g., Y1 receptor agonist and/or Y2 receptor antagonist)).

The number and frequency of doses may be determined based on the subject's response to administration of the composition, e.g., if one or more of the patient's symptoms improve and/or if the subject tolerates administration of the composition without adverse reaction; in some subjects, a single dose is sufficient, other subjects may receive a daily, several times a day, every other day, several times per week, weekly, biweekly, semi-weekly, or monthly administration of a composition containing NPY as described herein. The duration and frequency of treatment will depend upon the subject's response to treatment, i.e., if the subject's condition and/or one more symptoms of the mood and/or anxiety disorder improves.

In one example of a dosing regimen, an initial dose of NPY (or NPY analog, active fragment or conjugate of NPY or NPY analog, or other NPY receptor ligand (including, e.g., Y1 receptor agonist and/or Y2 receptor antagonist)) is used to treat a mood and/or anxiety disorder, followed by titration to a lower dose of NPY (or NPY analog, active fragment or conjugate of NPY or NPY analog, or other NPY receptor ligand (including, e.g., Y1 receptor agonist and/or Y2 receptor antagonist)) to maintain treatment of the PTSD. Such a regimen may be particularly useful, for example, to use a high dose of NPY (or NPY analog, active fragment or conjugate of NPY or NPY analog, or other NPY receptor ligand (including, e.g., Y1 receptor agonist and/or Y2 receptor antagonist)) to treat acute symptoms of the mood and/or anxiety disorder, followed by titrating to a lower dose of NPY (or NPY analog, active fragment or conjugate of NPY or NPY analog, or other NPY receptor ligand (including, e.g., Y1 receptor agonist and/or Y2 receptor antagonist)), to treat chronic symptoms of the mood and/or anxiety disorder.

In another example, a subject receives multiple administrations of NPY (or NPY analog, or other NPY receptor ligand (including, e.g., Y1 receptor agonist and/or Y2 receptor antagonist)) wherein the administration occurs on separate days. In some aspects at least two of the multiple administrations can occur on the same day.

In some aspects, a dose of NPY (or NPY analog, active fragment of NPY (or NPY analog, or other NPY receptor ligand (including, e.g., Y1 receptor agonist and/or Y2 receptor antagonist)) to treat a mood and/or anxiety disorder (e.g., Major Depressive Disorder, Panic Disorder, Specific phobia, Social Phobia, OCD, PTSD, acute stress disorder, and generalized anxiety disorder) is approximately 0.005 mg/kg to approximately 1 mg/kg body weight (mg/kg), approximately 0.005 mg/kg to approximately 0.9 mg/kg, approximately 0.005 mg/kg to approximately 0.8 mg/kg, approximately 0.005 mg/kg to approximately 0.75 mg/kg, approximately 0.005 mg/kg to approximately 0.6 mg/kg, approximately 0.005 mg/kg to approximately 0.5 mg/kg, approximately 0.005 mg/kg to approximately 0.4 mg/kg, approximately 0.005 mg/kg to approximately 0.3 mg/kg, approximately 0.005 mg/kg to approximately 0.2 mg/kg, 0.005 mg/kg to approximately 0.1 mg/kg, approximately 0.005 mg/kg to approximately 0.09 mg/kg, approximately 0.005 mg/kg to approximately 0.08 mg/kg, approximately 0.005 mg/kg to approximately 0.075 mg/kg, approximately 0.005 mg/kg to approximately 0.06 mg/kg, approximately 0.005 mg/kg to approximately 0.05 mg/kg, approximately 0.005 mg/kg to approximately 0.04 mg/kg, approximately 0.005 mg/kg to approximately 0.03 mg/kg, approximately 0.005 mg/kg to approximately 0.02 mg/kg, approximately 0.005 mg/kg to approximately 0.01 mg/kg, approximately 0.0075 mg/kg to approximately 1.0 mg/kg, approximately 0.0075 mg/kg to approximately 0.9 mg/kg, approximately 0.0075 mg/kg to approximately 0.8 mg/kg, approximately 0.0075 mg/kg to approximately 0.75 mg/kg, approximately 0.0075 mg/kg to approximately 0.6 mg/kg, approximately 0.0075 mg/kg to approximately 0.5 mg/kg, approximately 0.0075 mg/kg to approximately 0.4 mg/kg, approximately 0.0075 mg/kg to approximately 0.3 mg/kg, approximately 0.0075 mg/kg to approximately 0.2 mg/kg, 0.0075 mg/kg to approximately 0.1 mg/kg, approximately 0.0075 mg/kg to approximately 0.09 mg/kg, approximately 0.0075 mg/kg to approximately 0.08 mg/kg, approximately 0.0075 mg/kg to approximately 0.075 mg/kg, approximately 0.0075 mg/kg to approximately 0.06 mg/kg, approximately 0.0075 mg/kg to approximately 0.05 mg/kg, approximately 0.0075 mg/kg to approximately 0.04 mg/kg, approximately 0.0075 mg/kg to approximately 0.03 mg/kg, approximately 0.0075 mg/kg to approximately 0.02 mg/kg, approximately 0.0075 mg/kg to approximately 0.01 mg/kg, approximately 0.009 mg/kg to approximately 1.0 mg/kg, approximately 0.009 mg/kg to approximately 0.9 mg/kg, approximately 0.009 mg/kg to approximately 0.8 mg/kg, approximately 0.009 mg/kg to approximately 0.75 mg/kg, approximately 0.009 mg/kg to approximately 0.6 mg/kg, approximately 0.009 mg/kg to approximately 0.5 mg/kg, approximately 0.009 mg/kg to approximately 0.4 mg/kg, approximately 0.009 mg/kg to approximately 0.3 mg/kg, approximately 0.009 mg/kg to approximately 0.2 mg/kg, 0.009 mg/kg to approximately 0.1 mg/kg, approximately 0.009 mg/kg to approximately 0.09 mg/kg, approximately 0.009 mg/kg to approximately 0.08 mg/kg, approximately 0.009 mg/kg to approximately 0.075 mg/kg, approximately 0.009 mg/kg to approximately 0.06 mg/kg, approximately 0.009 mg/kg to approximately 0.05 mg/kg, approximately 0.009 mg/kg to approximately 0.04 mg/kg, approximately 0.009 mg/kg to approximately 0.03 mg/kg, approximately 0.009 mg/kg to approximately 0.02 mg/kg, approximately 0.009 mg/kg to approximately 0.01 mg/kg, approximately 0.05 mg/kg to approximately 1.0 mg/kg, approximately 0.05 mg/kg to approximately 0.9 mg/kg, approximately 0.05 mg/kg to approximately 0.8 mg/kg, approximately 0.05 mg/kg to approximately 0.75 mg/kg, approximately 0.05 mg/kg to approximately 0.6 mg/kg, approximately 0.05 mg/kg to approximately 0.5 mg/kg, approximately 0.05 mg/kg to approximately 0.4 mg/kg, approximately 0.05 mg/kg to approximately 0.3 mg/kg, approximately 0.05 mg/kg to approximately 0.2 mg/kg, 0.05 mg/kg to approximately 0.1 mg/kg, approximately 0.05 mg/kg to approximately 0.09 mg/kg, approximately 0.05 mg/kg to approximately 0.08 mg/kg, approximately 0.05 mg/kg to approximately 0.075 mg/kg, approximately 0.05 mg/kg to approximately 0.06 mg/kg, approximately 0.05 mg/kg to approximately 0.05 mg/kg, approximately 0.05 mg/kg to approximately 0.04 mg/kg, approximately 0.05 mg/kg to approximately 0.03 mg/kg, approximately 0.05 mg/kg to approximately 0.02 mg/kg, approximately 0.05 mg/kg to approximately 0.01 mg/kg, approximately 0.01 mg/kg to approximately 1.0 mg/kg, approximately 0.01 mg/kg to approximately 0.9 mg/kg, approximately 0.01 mg/kg to approximately 0.8 mg/kg, approximately 0.01 mg/kg to approximately 0.75 mg/kg, approximately 0.01 mg/kg to approximately 0.6 mg/kg, approximately 0.01 mg/kg to approximately 0.5 mg/kg, approximately 0.01 mg/kg to approximately 0.4 mg/kg, approximately 0.01 mg/kg to approximately 0.3 mg/kg, approximately 0.01 mg/kg to approximately 0.2 mg/kg, 0.01 mg/kg to approximately 0.1 mg/kg, approximately 0.01 mg/kg to approximately 0.09 mg/kg, approximately 0.01 mg/kg to approximately 0.08 mg/kg, approximately 0.01 mg/kg to approximately 0.075 mg/kg, approximately 0.01 mg/kg to approximately 0.06 mg/kg, approximately 0.01 mg/kg to approximately 0.05 mg/kg, approximately 0.01 mg/kg to approximately 0.04 mg/kg, approximately 0.01 mg/kg to approximately 0.03 mg/kg, approximately 0.01 mg/kg to approximately 0.02 mg/kg, approximately 0.006 mg/kg to approximately 1.0 mg/kg, 0.0075 mg/kg to approximately 1.0 mg/kg, 0.008 mg/kg to approximately 1.0 mg/kg, 0.009 mg/kg to approximately 1.0 mg/kg, 0.02 mg/kg to approximately 1.0 mg/kg, 0.03 mg/kg to approximately 1.0 mg/kg, 0.04 mg/kg to approximately 1.0 mg/kg, 0.05 mg/kg to approximately 1.0 mg/kg, 0.06 mg/kg to approximately 1.0 mg/kg, 0.075 mg/kg to approximately 1.0 mg/kg, 0.08 mg/kg to approximately 1.0 mg/kg, 0.09 mg/kg to approximately 1.0 mg/kg, 0.1 mg/kg to approximately 1.0 mg/kg, 0.2 mg/kg to approximately 1.0 mg/kg, 0.3 mg/kg to approximately 1.0 mg/kg, 0.4 mg/kg to approximately 1.0 mg/kg, 0.5 mg/kg to approximately 1.0 mg/kg, 0.6 mg/kg to approximately 1.0 mg/kg, 0.75 mg/kg to approximately 1.0 mg/kg, 0.8 mg/kg to approximately 1.0 mg/kg, 0.9 mg/kg to approximately 1.0 mg/kg, approximately 0.005 mg/kg to approximately 0.75 mg/kg, approximately 0.006 mg/kg to approximately 0.75 mg/kg, approximately 0.007 mg/kg to approximately 0.75 mg/kg, approximately 0.008 mg/kg to approximately 0.75 mg/kg, approximately 0.009 mg/kg to approximately 0.75 mg/kg, approximately 0.01 mg/kg to approximately 0.75 mg/kg, approximately 0.02 mg/kg to approximately 0.75 mg/kg, approximately 0.03 mg/kg to approximately 0.75 mg/kg, approximately 0.04 mg/kg to approximately 0.75 mg/kg, approximately 0.05 mg/kg to approximately 0.75 mg/kg, approximately 0.06 mg/kg to approximately 0.75 mg/kg, approximately 0.07 mg/kg to approximately 0.75 mg/kg, approximately 0.08 mg/kg to approximately 0.75 mg/kg, approximately 0.09 mg/kg to approximately 0.75 mg/kg, approximately 0.1 mg/kg to approximately 0.75 mg/kg, approximately 0.2 mg/kg to approximately 0.75 mg/kg, approximately 0.3 mg/kg to approximately 0.75 mg/kg, approximately 0.4 mg/kg to approximately 0.75 mg/kg, approximately 0.5 mg/kg to approximately 0.75 mg/kg, approximately 0.6 mg/kg to approximately 0.75 mg/kg, approximately 0.005 mg/kg to approximately 0.5 mg/kg, approximately 0.006 mg/kg to approximately 0.5 mg/kg, approximately 0.007 mg/kg to approximately 0.5 mg/kg, approximately 0.008 mg/kg to approximately 0.5 mg/kg, approximately 0.009 mg/kg to approximately 0.5 mg/kg, approximately 0.01 mg/kg to approximately 0.5 mg/kg, approximately 0.02 mg/kg to approximately 0.5 mg/kg, approximately 0.03 mg/kg to approximately 0.5 mg/kg, approximately 0.04 mg/kg to approximately 0.5 mg/kg, approximately 0.05 mg/kg to approximately 0.5 mg/kg, approximately 0.06 mg/kg to approximately 0.5 mg/kg, approximately 0.07 mg/kg to approximately 0.5 mg/kg, approximately 0.08 mg/kg to approximately 0.5 mg/kg, approximately 0.09 mg/kg to approximately 0.5 mg/kg, approximately 0.1 mg/kg to approximately 0.5 mg/kg, approximately 0.2 mg/kg to approximately 0.5 mg/kg, approximately 0.3 mg/kg to approximately 0.5 mg/kg, approximately 0.4 mg/kg to approximately 0.5 mg/kg, approximately 0.005 mg/kg to approximately 0.3 mg/kg, 0.006 mg/kg to approximately 0.3 mg/kg, approximately 0.007 mg/kg to approximately 0.3 mg/kg, approximately 0.008 mg/kg to approximately 0.3 mg/kg, approximately 0.009 mg/kg to approximately 0.3 mg/kg, 0.01 mg/kg to approximately 0.3 mg/kg, approximately 0.02 mg/kg to approximately 0.3 mg/kg, approximately 0.03 mg/kg to approximately 0.3 mg/kg, approximately 0.04 mg/kg to approximately 0.3 mg/kg, approximately 0.05 mg/kg to approximately 0.3 mg/kg, approximately 0.06 mg/kg to approximately 0.3 mg/kg, approximately 0.07 mg/kg to approximately 0.3 mg/kg, approximately 0.08 mg/kg to approximately 0.3 mg/kg, approximately 0.09 mg/kg to approximately 0.3 mg/kg, approximately 0.1 mg/kg to approximately 0.3 mg/kg, approximately 0.2 mg/kg to approximately 0.3 mg/kg, approximately 0.005 mg/kg to approximately 0.1 mg/kg, 0.006 mg/kg to approximately 0.1 mg/kg, approximately 0.007 mg/kg to approximately 0.1 mg/kg, approximately 0.008 mg/kg to approximately 0.1 mg/kg, approximately 0.009 mg/kg to approximately 0.1 mg/kg, 0.01 mg/kg to approximately 0.1 mg/kg, approximately 0.02 mg/kg to approximately 0.1 mg/kg, approximately 0.03 mg/kg to approximately 0.1 mg/kg, approximately 0.04 mg/kg to approximately 0.1 mg/kg, approximately 0.05 mg/kg to approximately 0.1 mg/kg, approximately 0.06 mg/kg to approximately 0.1 mg/kg, approximately 0.07 mg/kg to approximately 0.1 mg/kg, approximately 0.08 mg/kg to approximately 0.1 mg/kg, or approximately 0.09 mg/kg to approximately 0.1 mg/kg.

A subject (e.g., patient) suffering from a mood and/or anxiety disorder (e.g., Major Depressive Disorder, Panic Disorder, Specific phobia, Social Phobia, OCD, PTSD, acute stress disorder, and generalized anxiety disorder) may be administered (which includes self-administration) a dose of NPY (or NPY analog, active fragment or conjugate of NPY or NPY analog, or other NPY receptor ligand (including, e.g., Y1 receptor agonist and/or Y2 receptor antagonist)) of, for example, about 0.005 mg per kg of body weight (mg/kg), about 0.006 mg/kg, about 0.007 mg/kg, about 0.008 mg/kg, about 0.009 mg/kg, about 0.01 mg/kg, about 0.02 mg/kg, about 0.03 mg/kg, about 0.04 mg/kg, about 0.05 mg/kg, about 0.06 mg/kg, about 0.07 mg/kg, about 0.08 mg/kg, about 0.09 mg/kg, about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, or about 1.0 mg/kg.

In another embodiment, the total dose of NPY (or NPY analog, active fragment or conjugate of NPY or NPY analog, or other NPY receptor ligand (including, e.g., Y1 receptor agonist and/or Y2 receptor antagonist)) per nasal administration ranges from about 0.375 mg to about 75 mg. By way of non-limiting example, intranasal doses of NPY (or NPY analog, active fragment or conjugate of NPY or NPY analog, or other NPY receptor ligand (including, e.g., Y1 receptor agonist and/or Y2 receptor antagonist)) of 0.375 mg, 0.5 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 65 mg, 66 mg, 67 mg, 68 mg, 69 mg, 70 mg, 71 mg, 72 mg, 73 mg, 74 mg, and 75 mg are specifically contemplated.

In another embodiment, the total dose of NPY (or NPY analog, active fragment or conjugate of NPY or NPY analog, or other NPY receptor ligand (including, e.g., Y1 receptor agonist and/or Y2 receptor antagonist)) per day (e.g., by intranasal administration or other suitable route of administration) ranges from about 0.375 mg to about 200 mg. By way of non-limiting example, total per day dosages of NPY (or NPY analog, active fragment or conjugate of NPY or NPY analog, or other NPY receptor ligand (including, e.g., Y1 receptor agonist and/or Y2 receptor antagonist)) include: 0.375 mg, 0.5 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, 101 mg, 102 mg, 103 mg, 104 mg, 105 mg, 106 mg, 107 mg, 108 mg, 109 mg, 110 mg, 111 mg, 112 mg, 113 mg, 114 mg, 115 mg, 116 mg, 117 mg, 118 mg, 119 mg, 120 mg, 121 mg, 122 mg, 123 mg, 124 mg, 125 mg, 126 mg, 127 mg, 128 mg, 129 mg, 130 mg, 131 mg, 132 mg, 133 mg, 134 mg, 135 mg, 136 mg, 137 mg, 138 mg, 139 mg, 140 mg, 141 mg, 142 mg, 143 mg, 144 mg, 145 mg, 146 mg, 147 mg, 148 mg, 149 mg, 150 mg, 151 mg, 152 mg, 153 mg, 154 mg, 155 mg, 156 mg, 157 mg, 158 mg, 159 mg, 160 mg, 161 mg, 162 mg, 163 mg, 164 mg, 165 mg, 166 mg, 167 mg, 168 mg, 169 mg, 170 mg, 171 mg, 172 mg, 173 mg, 174 mg, 175 mg, 176 mg, 177 mg, 178 mg, 179 mg, 180 mg, 181 mg, 182 mg, 183 mg, 184 mg, 185 mg, 186 mg, 187 mg, 188 mg, 189 mg, 190 mg, 191 mg, 192 mg, 193 mg, 194 mg, 195 mg, 196 mg, 197 mg, 198 mg, 199 mg, and 200 mg.

In certain embodiments, the above total per day dosages may be administered to a subject as a single administration, or as multiple administrations per day. For multiple administrations, the total dosage may be divided equally per administration or unequally. In certain embodiments, a subject may be administered 1 dose, 2 doses, 3 doses, 4 doses, 5 doses, 6 doses or more of a NPY-containing composition described herein per day.

Preferably, the effective dose is titrated under the supervision of a physician or medical care provider, so that the optimum dose for the particular application is accurately determined. Thus, the present disclosure provides a dose suited to each individual subject (e.g., patient).

Once the dosage range is established, a further advantage of the presently disclosed compositions and methods of treatment is that the patient can administer NPY on an as-needed, dose-to-effect basis. Thus, the frequency of administration is under control of the subject. Yet another particular advantage is that intranasal administration of NPY is non-invasive, and facilitates NPY's crossing of the blood-brain barrier. The skilled artisan will appreciate, however, that other routes of administration, such as, but not limited to, those routes disclosed herein (e.g., sublingual, spinal injection, intravenous, etc.), are also contemplated.

As discussed above, in some embodiments, NPY (or NPY analog, active fragment or conjugate of NPY or NPY analog, or other NPY receptor ligand (including, e.g., Y1 receptor agonist and/or Y2 receptor antagonist)) is coadministered with a second therapeutic agent. In a preferred embodiment, a therapeutically effective amount of the second agent used for the treatment of a mood and/or anxiety disorder and/or a comorbid condition, is administered in conjunction with (or NPY analog, active fragment or conjugate of NPY or NPY analog, or other NPY receptor ligand (including, e.g., Y1 receptor agonist and/or Y2 receptor antagonist)). Typically the second therapeutic agent is administered in an amount such that the co-administration of (or NPY analog, active fragment or conjugate of NPY or NPY analog, or other NPY receptor ligand (including, e.g., Y1 receptor agonist and/or Y2 receptor antagonist)) and the second agent is therapeutically effective (i.e., effective for treating the mood and/or anxiety disorder and/or a comorbid condition).

### Kits

Kits comprising NPY (or NPY analog, active fragment or conjugate of NPY or NPY analog, or other NPY receptor ligand (including, e.g., Y1 receptor agonist and/or Y2 receptor antagonist)) are provided herein. In some embodiments, a kit comprises a composition comprising NPY (or NPY analog, active fragment or conjugate of NPY or NPY analog, or other NPY receptor ligand (including, e.g., Y1 receptor agonist and/or Y2 receptor antagonist)) for administration in a dosage of about 0.005 mg/kg to about 0.75 mg/kg, about 0.01 mg/kg to about 0.75 mg/kg, 0.05 mg/kg to about 0.75 mg/kg, 0.05 mg/kg to about 0.5 mg/kg, 0.01 mg/kg to about 0.5 mg/kg, 0.01 mg/kg to about 0.25 mg/kg, or 0.1 mg/kg to about 0.5 mg/kg. In other embodiments, a kit comprises a composition comprising NPY (or NPY analog, active fragment or conjugate of NPY or NPY analog, or other NPY receptor ligand (including, e.g., Y1 receptor agonist and/or Y2 receptor antagonist)) for administration in a dosage of about 0.05 mg/kg, or a dosage of about 1.0 mg/kg, or a dosage of about 0.5 mg/kg. The composition contained in the kit is preferably formulated for administration to humans, and more preferably for intranasal administration to humans. The kits contemplated herein can also comprise a carrier for delivering NPY (or NPY analog, active fragment or conjugate of NPY or NPY analog, or other NPY receptor ligand (including, e.g., Y1 receptor agonist and/or Y2 receptor antagonist)) intranasally, containing in close confinement therein one or more components, wherein: a) a first component contains NPY (or NPY analog, active fragment or conjugate of NPY or NPY analog, or other NPY receptor ligand (including, e.g., Y1 receptor agonist and/or Y2 receptor antagonist)) ; and b) a second component contains a second agent (e.g., psychotropic medication) useful in the treatment of a mood or anxiety disorder. In a further aspect, the second component can be lithium, a pharmaceutical antidepressant, an herbal antidepressant, ketamine, an anticonvulsant, a mood stabilizer, an antipsychotic agent, or a benzodiazepine. The kits contemplated herein can also comprise a device for administration of the composition(s) contained in the kits. For example, kits can comprise a device for intranasal administration of the composition(s) (e.g., composition comprising NPY (or NPY analog, fragment, conjugate, or other NPY receptor ligand (including, e.g., Y1 receptor agonist and/or Y2 receptor antagonist)) and, optionally, a second active agent (e.g. antidepressant, etc.). Preferred devices for intranasal delivery are those that deliver the active compound to the olfactory region of the nasal cavity (e.g., Optinose, Kurve® Technology, and similar devices).

Kits contemplated herein can further comprise instructions for use. Such instructions can, for example and not limited to, direct the optimal mode of administration, dosage and dosing regimen of the compound(s) contained in the kit.

### Modes of Administration

The compositions and formulations described herein (e.g., pharmaceutical formulation) comprising NPY (or NPY analog, active fragment or conjugate of NPY or NPY analog, or other NPY receptor ligand (including, e.g., Y1 receptor agonist and/or Y2 receptor antagonist)) may be for administration by oral (solid or liquid), parenteral (intramuscular, intraperitoneal, intravenous (IV) or subcutaneous injection), transdermal (either passively or using ionophoresis or electroporation), transmucosal (nasal, intranasal, vaginal, rectal, or sublingual), or inhalation routes of administration, or using bioerodible inserts and can be formulated in dosage forms appropriate for each route of administration. The most suitable route in any given case will depend on the particular host, and nature and severity of the conditions for which the active ingredient is being administered. The compositions may be conveniently presented in unit dosage form and prepared by any of the methods well known in the art of pharmacy, and using well known carriers and excipients.

In general, preparations according to this invention include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also optionally contain adjuvants, preserving, wetting, emulsifying, and dispersing agents. The pharmaceutical compositions may be sterilized by, for example, filtration through a bacteria retaining filter, by incorporating sterilizing agents into the compositions, by irradiating the compositions, or by heating the compositions. They can also be manufactured using sterile water, or some other sterile injectable medium, immediately before use.

### Intravenous Administration

A composition or formulation (e.g., pharmaceutical formulation) comprising NPY (or NPY analog, active fragment or conjugate of NPY or NPY analog, or other NPY receptor ligand (including, e.g., Y1 receptor agonist and/or Y2 receptor antagonist)) can be administered by intravenous (IV) administration. Compositions and formulations described herein may thus also be prepared in a formulation or pharmaceutical composition appropriate for IV administration. Compositions and formulations described herein can be admixed with a pharmaceutically acceptable carrier or excipient as described above. By way of example, the compositions and formulations described herein can be formulated in a saline solution for intravenous administration.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants, preserving, wetting, emulsifying, and dispersing agents. The pharmaceutical compositions may be sterilized by, for example, filtration through a bacteria retaining filter, by incorporating sterilizing agents into the compositions, by irradiating the compositions, or by heating the compositions. They can also be manufactured using sterile water, or some other sterile injectable medium, immediately before use.

### Nasal Administration

Most studies in humans have been carried out using a commercial nasal spray system with the subject sitting or standing using simple solution formulations. These studies, using gamma scintigraphy or PET scanning, have shown that such simple nasal sprays deposit the formulations anteriorly and high up in the nasal cavity and very little if any of the spray droplets reach the olfactory mucosa. As discussed above, it is particularly preferred that the compositions and formulations disclosed herein are delivered to the olfactory region of the nasal cavity to facilitate delivery of the drug directly to the brain. Thus, the present disclosure specifically contemplates devices that are designed for the delivery of pharmaceutical compositions and therapeutic formulations by intranasal delivery and delivery of the drug such that it reaches the olfactory region of the nasal cavity.

Thus, in certain embodiments, a composition or formulation (e.g., pharmaceutical formulation) comprising NPY (or NPY analog, active fragment or conjugate of NPY or NPY analog, or other NPY receptor ligand (including, e.g., Y1 receptor agonist and/or Y2 receptor antagonist)) is administered intranasally to a patient suffering from a mood and/or anxiety disorder. In a preferred embodiment, the composition or formulation comprising NPY (or an analog of NPY) is administered such that the composition or formulation contacts the olfactory region of the nasal cavity.

In one embodiment, the compositions and formulations described herein are administered to a patient as an aerosol spray for nasal inhalation. For example, NPY (or NPY analog, active fragment or conjugate of NPY or NPY analog, or other NPY receptor ligand (including, e.g., Y1 receptor agonist and/or Y2 receptor antagonist)), e.g., combined with a dispersing agent, a dispersant, a mucosal penetration enhancer, a mucoadhesive polymer and/or a vasoconstrictor, can be administered in an aerosol formulation optimized for intranasal administration (e.g., in a solution or suspension with a diluent).

Any form of aerosolization known in the art, including but not limited to spray bottles, nebulization, and atomization or pump aerosolization of a liquid formulation, can be used. However, particularly preferred are those that permit administration of the composition or formulation directly to the olfactory region of the nasal cavity, and preferably that also minimize entry of the drug into systemic circulation.

For intranasal administration, an exemplary delivery device is a small, hard bottle to which a metered dose sprayer is attached. In one embodiment, the metered dose is delivered by drawing the NPY (or NPY analog, active fragment or conjugate of NPY or NPY analog, or other NPY receptor ligand (including, e.g., Y1 receptor agonist and/or Y2 receptor antagonist)) solution into a chamber of defined volume, which chamber has an aperture dimensioned to aerosolize and aerosol formulation by forming a spray when a liquid in the chamber is compressed. The chamber is compressed to administer the NPY. In a specific embodiment, the chamber is a piston arrangement. Such devices are commercially available.

A plastic squeeze bottle with an aperture or opening dimensioned to aerosolize an aerosol formulation by forming a spray when squeezed. The opening is usually found in the top of the bottle, and the top is generally tapered to partially fit in the nasal passages for efficient administration of the aerosol formulation.

A preferred device for intranasal delivery of the compositions and formulations disclosed herein (e.g., comprising NPY, NPY analog, active fragment or conjugate of NPY or NPY analog, or other NPY receptor ligand (including, e.g., Y1 receptor agonist and/or Y2 receptor antagonist)) is Optinose, which is commercially available from OptiNose US Inc. (Yardley, PA). This device is particularly effective for administering a composition or formulation disclosed herein such that the composition or formulation is delivered to the olfactory region of the nasal cavity. In one embodiment, the drug (e.g., NPY, NPY analog or other derivative or NPY receptor agonist) can be formulated in a simple solution of phosphate buffered saline for delivery in the Optinose device. The Optinose nasal spray is a bi -directional system that directs the flow of the spray to pass from the opening of one nostril back through the nasal cavity and passes round past the soft palate, and out through the other nostril. The spray in the passage has close contact with the olfactory region. The formulation can be administered with the subject in a sitting position with the head tilted back.

Another preferred device for intranasal delivery is the Kurve® Technology device (Kurve Technology, Inc., Lynnwood, WA).

Also contemplated for administering an intranasal dose of the compositions and formulations disclosed herein are mucosal automation device that provide atomization of topical solution across the nasal and oropharyngeal mucous membranes that produce a typical particle size of 30 microns. For example, the LMA MAD Nasal™ device (LMA Company, San Diego, CA), which produces a typical particle size of 30 microns, has a system dead space of 0.09 mL, a tip diameter of about 3/16" (4mm), and an applicator length of about 1-3/4" (44mm) can be used. This device is particularly effective for administering a composition or formulation disclosed herein directly to the olfactory region of the nasal cavity.

In another embodiment, intranasal drug delivery is achieved by taking a solubilized medication (liquid form) and dripping it into the nose a few drops at a time, allowing it to run down onto the nasal mucosa. This can be done using, e.g., a syringe.

### Spinal Administration

A composition or formulation (e.g., pharmaceutical formulation) comprising NPY (or NPY analog, active fragment or conjugate of NPY or NPY analog, or other NPY receptor ligand (including, e.g., Y1 receptor agonist and/or Y2 receptor antagonist)) can be administered by injection into the spinal fluid (e.g., injection into the cerebrospinal fluid).

### Mood and Anxiety Disorders

Mood and anxiety disorders that can be treated according to the methods disclosed herein are described below.

### Major Depressive Disorder

Major Depressive Disorder is characterized by one or more Major Depressive Episodes (i.e., at least 2 weeks of depressed mood or loss of interest accompanied by at least four additional symptoms of depression). The essential feature of a Major Depressive Episode is a period of at least 2 weeks during which there is either depressed mood or the loss of interest or pleasure in nearly all activities. The individual typically experiences additional symptoms such as any of changes in appetite or weight, sleep, and psychomotor activity; decreased energy; feelings of worthlessness or guilt; difficulty thinking, concentrating, or making decisions; or recurrent thoughts of death or suicidal ideation, plans, or attempts. The symptoms typically persist for most of the day, nearly every day, for at least 2 consecutive weeks. The episode is accompanied by clinically significant distress or impairment in social, occupational, or other important areas of functioning. The most common sleep disturbance associated with a Major Depressive Episode is insomnia.

### Bipolar Disorder

Bipolar I Disorder is characterized by one or more Manic or Mixed Episodes, usually accompanied by Major Depressive Episodes. Bipolar II Disorder is characterized by one or more Major Depressive Episodes accompanied by at least one Hypomanic Episode. The method disclosed herein can be used to treat the depressive aspect of Bipolar disorders.

### Panic Disorder

Panic Disorder Without Agoraphobia is characterized by recurrent unexpected Panic Attacks about which there is persistent concern. Panic Disorder With Agoraphobia is characterized by both recurrent unexpected Panic Attacks and Agoraphobia.

A Panic Attack is a discrete period in which there is the sudden onset of intense apprehension, fearfulness, or terror, often associated with feelings of impending doom. During these attacks, symptoms such as shortness of breath, palpitations, chest pain or discomfort, choking or smothering sensations, and fear of "going crazy" or losing control are present. Symptoms can be somatic or cognitive in nature and include palpitations, sweating, trembling or shaking, sensations of shortness of breath or smothering, feeling of choking, chest pain or discomfort, nausea or abdominal distress, dizziness or lightheadedness, derealization or depersonalization, fear of losing control or "going crazy," fear of dying, paresthesias, and chills or hot flushes. The attack has a sudden onset and builds to a peak rapidly (usually in 10 minutes or less) and is often accompanied by a sense of imminent danger or impending doom and an urge to escape. The anxiety that is characteristic of a Panic Attack can be differentiated from generalized anxiety by its discrete, almost paroxysmal, nature and its typically greater severity. Attacks that meet all other criteria but that have fewer than 4 somatic or cognitive symptoms are referred to as limited-symptom attacks.

Agoraphobia is anxiety about, or avoidance of, places or situations from which escape might be difficult (or embarrassing) or in which help may not be available in the event of having a Panic Attack or panic-like symptoms.

### Specific Phobia

Specific Phobia is characterized by clinically significant anxiety provoked by exposure to a specific feared object or situation, often leading to avoidance behavior. The essential feature of Specific Phobia is marked and persistent fear of clearly discernible, circumscribed objects or situations. Exposure to the phobic stimulus almost invariably provokes an immediate anxiety response.

### Social Phobia (Social Anxiety Disorder)

Social Phobia is characterized by clinically significant anxiety provoked by exposure to certain types of social or performance situations, often leading to avoidance behavior. The essential feature of Social Phobia is a marked and persistent fear of social or performance situations in which embarrassment may occur. Exposure to the social or performance situation almost invariably provokes an immediate anxiety response. Most often, the social or performance situation is avoided, although it is sometimes endured with dread.

### Obsessive-Compulsive Disorder

Obsessive-Compulsive Disorder (OCD) is characterized by obsessions (which cause marked anxiety or distress) and/or by compulsions (which serve to neutralize anxiety). The essential features of OCD are recurrent obsessions or compulsions (Criterion A) that are severe enough to be time consuming (i.e., they take more than 1 hour a day) or cause marked distress or significant impairment (Criterion C). At some point during the course of the disorder, the person has recognized that the obsessions or compulsions are excessive or unreasonable (Criterion B). Obsessions are persistent ideas, thoughts, impulses, or images that are experienced as intrusive and inappropriate and that cause marked anxiety or distress. The most common obsessions are repeated thoughts about contamination (e.g., becoming contaminated by shaking hands), repeated doubts (e.g., wondering whether one has performed some act such as having hurt someone in a traffic accident or having left a door unlocked), a need to have things in a particular order (e.g., intense distress when objects are disordered or asymmetrical), aggressive or horrific impulses (e.g., to hurt one's child or to shout an obscenity in church), and sexual imagery (e.g., a recurrent pornographic image). The thoughts, impulses, or images are not simply excessive worries about real-life problems (e.g., concerns about current ongoing difficulties in life, such as financial, work, or school problems) and are unlikely to be related to a real-life problem. Compulsions are either clearly excessive or are not connected in a realistic way with what they are designed to neutralize or prevent. The most common compulsions involve washing and cleaning, counting, checking, requesting or demanding assurances, repeating actions, and ordering.

### PTSD

Posttraumatic Stress Disorder is characterized by the reexperiencing of an extremely traumatic event accompanied by symptoms of increased arousal and by avoidance of stimuli associated with the trauma. The essential feature of Posttraumatic Stress Disorder is the development of characteristic symptoms following exposure to an extreme traumatic stressor involving direct personal experience of an event that involves actual or threatened death or serious injury, or other threat to one's physical integrity; or witnessing an event that involves death, injury, or a threat to the physical integrity of another person; or learning about unexpected or violent death, serious harm, or threat of death or injury experienced by a family member or other close associate (Criterion A1). The person's response to the event must involve intense fear, helplessness, or horror (or in children, the response must involve disorganized or agitated behavior) (Criterion A2). The characteristic symptoms resulting from the exposure to the extreme trauma include persistent reexperiencing of the traumatic event (Criterion B), persistent avoidance of stimuli associated with the trauma and numbing of general responsiveness (Criterion C), and persistent symptoms of increased arousal (Criterion D). The full symptom picture must be present for more than 1 month (Criterion E), and the disturbance must cause clinically significant distress or impairment in social, occupational, or other important areas of functioning (Criterion F).

Traumatic events that are experienced directly include, but are not limited to, military combat, violent personal assault (sexual assault, physical attack, robbery, mugging), being kidnapped, being taken hostage, terrorist attack, torture, incarceration as a prisoner of war or in a concentration camp, natural or manmade disasters, severe automobile accidents, or being diagnosed with a life-threatening illness. For children, sexually traumatic events may include developmentally inappropriate sexual experiences without threatened or actual violence or injury. Witnessed events include, but are not limited to, observing the serious injury or unnatural death of another person due to violent assault, accident, war, or disaster or unexpectedly witnessing a dead body or body parts. Events experienced by others that are learned about include, but are not limited to, violent personal assault, serious accident, or serious injury experienced by a family member or a close friend; learning about the sudden, unexpected death of a family member or a close friend; or learning that one's child has a life-threatening disease. The disorder may be especially severe or long lasting when the stressor is of human design (e.g., torture, rape). The likelihood of developing this disorder may increase as the intensity of and physical proximity to the stressor increase.

Typically, the traumatic event is persistently re-experienced in one or more of the following ways: recurrent and intrusive distressing recollections of the event, including images, thoughts, or perceptions, recurrent distressing dreams of the event, acting or feeling as if the traumatic event were recurring (includes a sense of reliving the experience, illusions, hallucinations, and dissociative flashback episodes, including those that occur on awakening or when intoxicated), intense psychological distress at exposure to internal or external cues that symbolize or resemble an aspect of the traumatic event, physiological reactivity on exposure to internal or external cues that symbolize or resemble an aspect of the traumatic event. An individual suffering from PTSD also has persistent avoidance of stimuli associated with the trauma and numbing of general responsiveness (not present before the trauma), as indicated by 3 or more of the following: efforts to avoid thoughts, feelings, or conversations associated with the trauma, efforts to avoid activities, places, or people that arouse recollections of the trauma, inability to recall an important aspect of the trauma, significantly diminished interest or participation in significant activities, feeling of detachment or estrangement from others, restricted range of affect (e.g., unable to have loving feelings), sense of a foreshortened future (e.g., does not expect to have a career, marriage, children, or a normal life span), persistent symptoms of increased arousal (not present before the trauma), as indicated by 2 or more of the following: difficulty falling or staying asleep, irritability or outbursts of anger, difficulty concentrating, hypervigilance, exaggerated startle response. The disturbance, which has lasted for at least a month, causes clinically significant distress or impairment in social, occupational, or other important areas of functioning.

### Acute Stress Disorder

Acute Stress Disorder is characterized by symptoms similar to those of Posttraumatic Stress Disorder that occur immediately in the aftermath of an extremely traumatic event. The essential feature of Acute Stress Disorder is the development of characteristic anxiety, dissociative, and other symptoms that occurs within 1 month after exposure to an extreme traumatic stressor (Criterion A). Either while experiencing the traumatic event or after the event, the individual typically has at least three of the following dissociative symptoms: a subjective sense of numbing, detachment, or absence of emotional responsiveness; a reduction in awareness of his or her surroundings; derealization; depersonalization; or dissociative amnesia (Criterion B). Following the trauma, the traumatic event is persistently reexperienced (Criterion C), and the individual displays marked avoidance of stimuli that may arouse recollections of the trauma (Criterion D) and has marked symptoms of anxiety or increased arousal (Criterion E). The symptoms must cause clinically significant distress, significantly interfere with normal functioning, or impair the individual's ability to pursue necessary tasks (Criterion F). The disturbance lasts for a minimum of 2 days and a maximum of 4 weeks after the traumatic event (Criterion G); if symptoms persist beyond 4 weeks, the diagnosis of Posttraumatic Stress Disorder may be applied. The symptoms are not due to the direct physiological effects of a substance (i.e., a drug of abuse, a medication) or a general medical condition, are not better accounted for by Brief Psychotic Disorder, and are not merely an exacerbation of a preexisting mental disorder (Criterion H).

### Generalized Anxiety Disorder

Generalized Anxiety Disorder is characterized by at least 6 months of persistent and excessive anxiety and worry. The essential feature of Generalized Anxiety Disorder is excessive anxiety and worry (apprehensive expectation), occurring more days than not for a period of at least 6 months, about a number of events or activities (Criterion A). The individual finds it difficult to control the worry (Criterion B). The anxiety and worry are accompanied by at least three additional symptoms from a list that includes restlessness, being easily fatigued, difficulty concentrating, irritability, muscle tension, and disturbed sleep (only one additional symptom is required in children) (Criterion C). The focus of the anxiety and worry is not confined to features of another Axis I disorder such as having a Panic Attack (as in Panic Disorder), being embarrassed in public (as in Social Phobia), being contaminated (as in Obsessive-Compulsive Disorder), being away from home or close relatives (as in Separation Anxiety Disorder), gaining weight (as in Anorexia Nervosa), having multiple physical complaints (as in Somatization Disorder), or having a serious illness (as in Hypochondriasis), and the anxiety and worry do not occur exclusively during Posttraumatic Stress Disorder (Criterion D). Although individuals with Generalized Anxiety Disorder may not always identify the worries as "excessive," they report subjective distress due to constant worry, have difficulty controlling the worry, or experience related impairment in social, occupational, or other important areas of functioning (Criterion E). The disturbance is not due to the direct physiological effects of a substance (i.e., a drug of abuse, a medication, or toxin exposure) or a general medical condition and does not occur exclusively during a Mood Disorder, a Psychotic Disorder, or a Pervasive Developmental Disorder (Criterion F).

### Methods of Treating and Preventing Mood and Anxiety Disorders

Methods for treating a human patient with a mood disorder (e.g., Major Depressive Disorder and the depressive aspect of bipolar disorder) and/or anxiety disorder (e.g., Panic Disorder, Specific phobia, Social Phobia, obsessive-compulsive disorder, PTSD, acute stress disorder, generalized anxiety disorder) are provided herein. The methods include administering NPY (or NPY analog, active fragment or conjugate of NPY or NPY analog, or other NPY receptor ligand (including, e.g., Y1 receptor agonist and/or Y2 receptor antagonist)) to eliminate at least one symptom of the mood and/or anxiety disorder in the patient. In certain embodiments, administering NPY (or NPY analog, active fragment or conjugate of NPY or NPY analog, or other NPY receptor ligand (including, e.g., Y1 receptor agonist and/or Y2 receptor antagonist)) to a subject treats at least one symptom of more than one mood disorder and/or anxiety disorder.

In a specific embodiment, methods for treating a human patient with PTSD are directed to using NPY (or NPY analog, active fragment or conjugate of NPY or NPY analog, or other NPY receptor ligand (including, e.g., Y1 receptor agonist and/or Y2 receptor antagonist)) to eliminate at least one symptom of PTSD in the patient.

In other embodiments, the present disclosure also contemplates the prophylactic use of the NPY (or NPY analog, active fragment or conjugate of NPY or NPY analog, or other NPY receptor ligand (including, e.g., Y1 receptor agonist and/or Y2 receptor antagonist)) -containing compositions and formulations disclosed herein. For example, in certain embodiments, presently provided are methods for inhibiting development of a mood and/or anxiety disorder in a human patient which comprises intranasally administering to a subject in need of such inhibiting a composition comprising a therapeutically effective amount of NPY (or NPY analog, active fragment or conjugate of NPY or NPY analog, or other NPY receptor ligand (including, e.g., Y1 receptor agonist and/or Y2 receptor antagonist)) for inhibiting the development of the mood and/or anxiety disorder , wherein the therapeutically effective amount is a dosage range of about 0.005 mg/kg to about 1 mg/kg.

In another embodiment, provided herein is a method for treating a human patient for a mood or anxiety disorder which comprises intranasally administering to a human patient in need of such treatment a composition comprising a therapeutically effective dose of NPY for reducing or eliminating one or more symptoms of the mood or anxiety disorder. The therapeutically effective dose can be in the range of about 1 mg to about 20 mg of NPY. In some embodiments, the therapeutically effective dose is administered to the patient at least once a day. In some embodiments, the dose is in the range of about 1 mg to about 100 mg, about 1 mg to about 90 mg, about 1 mg to about 80 mg, about 1 mg to about 70 mg, about 1 mg to about 60 mg, about 1 mg to about 50 mg, about 1 mg to about 40 mg, about 1 mg to about 30 mg, about 1 mg to about 20 mg, about 1 mg to about 10 mg, or about 1 mg to about 5 mg. In some embodiments, a total dose is administered to the patient in a range of between about 1 and about 100 mg/day of NPY. In some embodiments, the total dose is in the range of about 1 mg to about 100 mg, about 1 mg to about 90 mg, about 1 mg to about 80 mg, about 1 mg to about 70 mg, about 1 mg to about 60 mg, about 1 mg to about 50 mg, about 1 mg to about 40 mg, about 1 mg to about 30 mg, about 1 mg to about 20 mg, about 1 mg to about 10 mg, or about 1 mg to about 5 mg.

In a specific embodiment, NPY is administered prophylactically to inhibit the development of one more symptoms of PTSD. Thus, in certain embodiments, for example, a subject may be administered (including self-administered) a composition or formulation comprising a therapeutically effective amount of NPY (or NPY analog, active fragment or conjugate of NPY or NPY analog, or other NPY receptor ligand (including, e.g., Y1 receptor agonist and/or Y2 receptor antagonist)) prior to a situation in which the subject is likely to be exposed to traumatic stress, such as for early responders and military personnel, immediately after exposure to traumatic stress, and/or when an individual feels that his or her PTSD symptoms are likely to appear.

In certain embodiments, for the treatment of a mood and/or anxiety disorder (e.g., treating at least one symptom of the mood and/or anxiety disorder), one or more symptoms of the mood and/or anxiety disorder are alleviated within 2 hours of administration of the NPY (or NPY analog, active fragment or conjugate of NPY or NPY analog, or other NPY receptor ligand (including, e.g., Y1 receptor agonist and/or Y2 receptor antagonist)). As disclosed herein, symptoms of the mood and/or anxiety disorder may be alleviated concomitant with administration of NPY (or NPY analog, active fragment or conjugate of NPY or NPY analog, or other NPY receptor ligand (including, e.g., Y1 receptor agonist and/or Y2 receptor antagonist)).

Treatment of the mood and/or anxiety disorder may be achieved following administration of a single dose of a composition comprising NPY (or NPY analog, active fragment or conjugate of NPY or NPY analog, or other NPY receptor ligand (including, e.g., Y1 receptor agonist and/or Y2 receptor antagonist)). Alternatively, multiple doses of the composition may be administered. Administration of a single dose of the composition can be sufficient to alleviate the effects of the mood and/or anxiety disorder for 1, day, 2 days, 3 days, 4 days, 5 days, 6 days, 7 days, 2 weeks and in some cases, longer.

In some embodiments, one or more secondary active agents, such as one of those described above, are coadministered to a patient in a combination therapy. As discussed above, "coadministered" does not necessarily mean that the drugs and/or therapies are administered in a combined form (i.e., they may be administered separately (i.e., as separate compositions or formulations) or together (the same formulation or composition) to the same or different sites at the same or different times). In one embodiment, a patient suffering from a mood and/or anxiety disorder is administered a combination therapy comprising NPY and an antidepressant agent (e.g., SSRI or ketamine (see, U.S. Patent Publication No. 2007/0287753). Thus, a composition comprising NPY (or NPY analog, active fragment or conjugate of NPY or NPY analog, or other NPY receptor ligand (including, e.g., Y1 receptor agonist and/or Y2 receptor antagonist)) may also be used to treat a mood and/or anxiety disorder in combination with a second agent.

Such second agents are described in detail above, with respect to formulations of NPY, and include but are not limited to, e.g., antidepressants: e.g., biogenic amine non-selective reuptake inhibitors, e.g., tricyclic antidepressants like imipramine; serotonin selective reuptake inhibitors like fluoxetine (Prozac); diazepam; monoamine oxidase (MAO) inhibitors, e.g., iproniazid, clorgyline, phenelzine, tranylcypromine, and isocarboxazid; biogenic amine uptake blockers, e.g., tricyclic antidepressants such as desipramine, imipramine and amitriptyline; atypical antidepressants such as mirtazapine, nefazodone, bupropion; serotonin reuptake inhibitors e.g., fluoxetine, venlafaxine, and duloxetine; antipsychotic drugs such as phenothiazine derivatives (e.g., chlorpromazine (thorazine) and trifluopromazine)), butyrophenones (e.g., haloperidol (Haldol)), thioxanthene derivatives (e.g., chlorprothixene), S and dibenzodiazepines (e.g., clozapine); benzodiazepines; dopaminergic agonists and antagonists e.g., L-DOPA, cocaine, amphetamine, a-methyl-tyrosine, reserpine, tetrabenazine, benztropine, pargyline; noradrenergic agonists and antagonists e.g., clonidine, phenoxybenzamine, phentolamine, tropolone, and ketamine.

A second agent may be administered as an adjunct therapy to the composition. For example, and without limitation, an individual suffering from a mood and/or anxiety disorder may be treated concurrently with a composition comprising NPY (or NPY analog, active fragment or conjugate of NPY or NPY analog, or other NPY receptor ligand (including, e.g., Y1 receptor agonist and/or Y2 receptor antagonist)) and the second agent. Alternatively, an individual suffering from a mood and/or anxiety disorder may be treated initially the composition, followed by cessation of treatment with the NPY-containing composition and initiation of treatment with a second agent. Such a treatment may be warranted, for example, if a patient exhibits unwanted side effects or reduced response to the composition (containing NPY) treatment. Alternatively, in certain embodiments, a composition comprising NPY (or NPY analog, active fragment or conjugate of NPY or NPY analog, or other NPY receptor ligand (including, e.g., Y1 receptor agonist and/or Y2 receptor antagonist)) is used as an initial treatment for the mood and/or anxiety disorder, e.g., by administration of one, two or three doses, and a second agent is administered to prolong the effect of the composition on reducing one or more of the symptoms of the mood and/or anxiety disorder, or alternatively, to boost the effect of the composition for reducing the mood and/or anxiety disorder. A person of ordinary skill in the art will recognize that other variations of the presented schemes are possible, e.g., initiating treatment of a patient having a mood and/or anxiety disorder with the composition (containing, e.g., NPY), followed by a period wherein the patient is treated with a second agent as adjunct therapy to treatment with the composition, followed by cessation of treatment with the composition.

Moreover, when administered separately from the NPY-containing composition, the second agent can be administered by any suitable route of administration. For example a second agent can be administered by a suitable "systemic" route of administration, such as, but not limited to, parenteral, oral, transdermal, transmucosal, intranasal, and buccal administration. Parenteral administration includes subcutaneous, intravenous, intramuscular, intra-articular, intra-synovial, intra-arteriole, intradermal, intraperitoneal, intraventricular, intrasternal, intrathecal, intrahepatic, intralesional, and intracranial administration.

A composition can also be administered with other treatment modalities with antidepressant effects such as, e.g., electro-convulsive treatment (ECT); light therapy psychotherapy e.g., cognitive or interpersonal therapy.

In certain embodiments, it can be determined whether a method for treating a mood and/or anxiety disorder, as disclosed herein, is effective, by determining whether one or more symptoms of the mood and/or anxiety disorder is reduced or eliminated. Psychiatric evaluations of a patient being treated with a therapy disclosed herein can be conducted to determine whether the therapy (e.g., intranasal administration of NPY (or NPY analog, active fragment or conjugate of NPY or NPY analog, or other NPY receptor ligand (including, e.g., Y1 receptor agonist and/or Y2 receptor antagonist)) is effective. In certain embodiments, the psychiatric evaluation may be carried out before treatment, at the time of treatment, during treatment, and/or after treatment. When the psychiatric evaluation is carried out both before treatment and after (and/or during) treatment with a therapy as disclosed herein, the results of the evaluation before treatment can provide a baseline for comparison to the results of the evaluation during and/or after treatment. However, in some embodiments, psychiatric evaluation is conducted only after treatment.

The methods for using NPY (or NPY analog, active fragment or conjugate of NPY or NPY analog, or other NPY receptor ligand (including, e.g., Y1 receptor agonist and/or Y2 receptor antagonist)) to treat mood and/or anxiety disorder described herein may also be used to treat a co-morbid conditions that respond to NPY (or NPY analog, active fragment or conjugate of NPY or NPY analog, or other NPY receptor ligand (including, e.g., Y1 receptor agonist and/or Y2 receptor antagonist)). Major depressive disorder, for example, exhibits co-morbidity with PTSD. In certain embodiments, for example, the methods described herein can thus be used to treat co-morbid PTSD and major depressive disorder.

### Measures

Mood and anxiety disorders are diagnosed by the history, presenting symptoms and mental status exam of a patient. To determine whether one or more symptoms of a patient's mood and/or anxiety disorder(s) has/have been treated, both clinician-administered questionnaires and validated self-report instruments can be used, with the aim of measuring baseline symptomatology as well as drug actions on (1) the overall severity of the disorder, (2) the core symptoms of the mood or anxiety disorder, and (3) depressed mood. A patient's physician will understand how to make such determination, and the following measures are described as non-limiting examples only, and are not intended nor need to be exhaustive of all possible measures that can be performed.

The Structured Clinical Interview for DSM-IV Axis I Disorders, Patient Edition (SCID-P) is a semi-structured interview that provides probe questions as well as follow-up questions to be asked by the clinician to assist in diagnosis. First et al., Structured Clinical Interview for DSM-IV TR Axis I Disorders, Research Version, Patient Edition (SCID-I/P). New York: New York State Psychiatric Institute, Biometrics Research; 2001. It includes an overview to obtain information about demographics, work, chief complaint, history of present illness, past history, treatment history, and current functioning. The main body of SCID-P includes 9 modules that are designed to diagnose 51 mental illnesses in all.

The Clinician-Administered PTSD Scale (CAPS) is a 30-item structured interview that corresponds to the DSM-IV criteria for PTSD. The CAPS can be used to make a current (past month) or lifetime diagnosis of PTSD or to assess symptoms over the past week. CAPS is described, e.g., in Blake, D. D., F. W. Weathers, et al. (1995). "The development of a Clinician-Administered PTSD Scale." Journal of traumatic stress 8(1): 75-90.

The Hamilton Psychiatric Rating Scale for Anxiety (HAM-A) is a widely used observational rating measure of anxiety severity. The scale consists of 14 items. Each item is rated on a scale of 0 to 4. This scale will be administered to assess the severity of anxiety and its improvement during the course of treatment. The HAM-A total score is the sum of the 14 items and the score ranges from 0 to 56. Hamilton M. The Assessment of Anxiety-States by Rating. Br J Med Psychol. 1959;32(1):50-55.

The Montgomery-Asberg Depression Rating Scale (MADRS) is a 10-item instrument used for the evaluation of depressive symptoms in adults and for the assessment of any changes to those symptoms. Montgomery S.A., et al., A new depression scale designed to be sensitive to change. Br J Psychiatry. 1979 Apr;134:382-389. Each of the 10 items is rated on a scale of 0 to 6, with differing descriptors for each item. These individual item scores are added together to form a total score, which can range between 0 and 60 points. The estimated time to administer this scale is 20 minutes. Inter-rater reliability of the scale is high and scores correlate significantly with those of the HAM-D. On the infusion days a modified MADRS will be used that will exclude the sleep and appetite items.

The Patient Rated Inventory of Side Effects (PRISE) Adverse Event Visit Checklist is a clinician-administered questionnaire used to qualify side effects by identifying and evaluating the tolerability of each symptom.

The Beck Depression Inventory (Second Edition) (BDI-II) is a 21-item self-report instrument intended to assess the existence and severity of symptoms of depression. The questionnaire is designed for individuals aged 13 and over, and is composed of items relating to symptoms of depression such as hopelessness and irritability, cognitions such as guilt or feelings of being punished, as well as physical symptoms such as fatigue, weight loss, and lack of interest in sex. See, e.g., Beck, A. T., R. A. Steer, et al. (1996). "Comparison of Beck Depression Inventories -IA and -II in psychiatric outpatients." Journal of personality assessment 67(3): 588-597.

The Beck Anxiety Inventory (BAI) is a 21-question multiple-choice self-report inventory that is used for measuring the severity of an individual's anxiety. The BAI consists of twenty-one questions about how the subject has been feeling in the last week, expressed as common symptoms of anxiety (such as numbness, hot and cold sweats, or feelings of dread). See, e.g., Beck, A. T. and R. A. Steer (1993). Beck Anxiety Inventory Manual. San Antonio, TX, The Psychological Corporation Harcourt Brace & Company.

The Profile of Mood States (POMS) provides a rapid, economical method of assessing transient, fluctuating active mood states. It is an ideal instrument for measuring and monitoring treatment change in clinical, medical, and addiction counseling centers. It is also well-suited to clinical drug trials because its sensitivity to change allows accurate documentation of the effects of drugs on mood state. See, e.g., McNair, D. M., M. Lorr, et al. (1982). Manual for the Profile Of Mood States, bipolar form (POMS-BI). San Diego, CA, Educational and Industrial Testing Devices; J Natl Cancer Inst 101(10): 708-720.

The Appetite Scale is a visual analog scale to measure level of hunger. See, e.g., Bond, A. and M. Lader (1974). "The use of analogue scales in rating subjective feelings." British Journal of Medical Psychology 47(3): 211-218.

The Clinical Global Impression (CGI) scale assesses treatment response in psychiatric patients. The administration time is 2 minutes. This scale consists of three items: Severity of Illness (item 1); Global Improvement (item 2); and Efficacy Index (item 3). Item 1 is rated on a seven-point scale (1 = normal, 7 = among the most extremely ill patients) as is item 2 (1 = very much improved, 7 = very much worse). Each includes an additional response of "not assessed." Item 3 is rated on a four-point scale (from "none" to "outweighs therapeutic effect"). Only items 1 (CGI-Severity or CGI-S) and 2 (CGI-Improvement or CGI-I) will be used.

The Impact of Events Scale (IES) is one of the most widely used self-report measures of stress reactions to traumatic events. Horowitz M, Wilner N, Alvarez W. Impact of Event Scale: a measure of subjective stress. Psychosom Med. 1979 May;41(3):209-218. It measures both intrusion and avoidance. A 2002 review that evaluated its psychometric properties revealed high internal consistencies for both subscales (intrusion: mean ? = 0.86; avoidance: mean ? = 0.82), adequate test-retest reliability for intervals of >1 year, and good internal and external validity. Sundin EC, Horowitz MJ. Impact of Event Scale: psychometric properties. Br J Psychiatry. 2002 Mar;180:205-209. Correlations between IES subscales and PTSD diagnosis assessed with the CAPS are high (>0.75). Id. The IES is considered particularly useful as a measure of the intrusive and avoidant processes that mediate between the experience of trauma and subsequent adjustment. Joseph S. Psychometric evaluation of Horowitz's Impact of Event Scale: a review. J Trauma Stress. 2000 Jan;13(1):101-113. The total score can range from 0 to 75.

The Quick Inventory of Depressive Symptomatology, Self Report (QIDS-SR) is a 16-item self rated instrument designed to assess the severity of depressive symptoms present in the past seven days. Rush AJ, Trivedi MH, Ibrahim HM et al. The 16-Item quick inventory of depressive symptomatology (QIDS), clinician rating (QIDS-C), and self-report (QIDS-SR): a psychometric evaluation in patients with chronic major depression. Biol Psychiatry. 2003;54(5):573-583. The 16 items cover the nine symptom domains of major depression, and are rated on a scale of 0-3. Total score ranges from 0 to 27, with ranges of 0-5 (normal), 6-10 (mild), 11-15 (moderate), 16-20 (moderate to severe), and 21+ (severe).

The Childhood Trauma Questionnaire (CTQ) is a 28-item self-report instrument that assesses childhood trauma in the following areas: physical, sexual and emotional abuse and physical and emotional neglect. Bernstein DP, Stein JA, Newcomb MD et al. Development and validation of a brief screening version of the Childhood Trauma Questionnaire. Child Abuse Negl. 2003 Feb;27(2): 169-190. Each item is rated on a scale of 1 (never true) to 5 (very often true). The 5 subscales are then totaled, with scores ranging from 5-25 for each traumatic category.

The Visual Analogue Scales (VAS) are used to assess subjective state changes. They are 100-mm horizontal lines marked proportionately to the perceived intensity of the subjective experience (0=not at all, to 10=extremely) for the following states: anxious, depressed, drowsy, high, hungry, and nauseous. See, e.g., Bond, A. and M. Lader (1974) *supra.*

The State-Trait Anxiety Inventory (STAI) is a self-report instrument that differentiates between the temporary condition of state anxiety and the longstanding quality of trait anxiety. With a fifth grade reading level, the STAI is suitable for individuals who are 15 years old and older. See, e.g., Spielberger, C. D. (1985). "Assessment of state and trait anxiety: Conceptual and methodological issues." Southern Psychologist Vol 2(4), 6-16.

The Clinician-Administered Dissociative States Scale (CADSS) is used to measure dissociative effects during the infusions. Bremner JD, et al., Measurement of Dissociative States with the Clinician-Administered Dissociative States Scale (CADSS). J Trauma Stress. 1998; 1 1(1): 125-136. The scale includes 19 questions and 8 observer ratings scored from 0 (not at all) to 4 (extremely). The CADSS measures impairment in body perception, environmental perception, time perception, memory impairment, and feelings of unreality.

The Sheehan Disability Scale (SDS) is the most frequently used self-report disability measure. It has demonstrated sensitivity to impairment and changes as a result of treatment across a wide range of psychiatric disorders. The SDS asks only about current levels of impairment, providing no indication of whether the person has done better or worse in the past, thus making it a reasonable short-term outcome measure that is un-confounded by historical impressions. The dependent variable is the total score, which is based on the sum of three 10-point items (work, social life, and family life), with higher scores reflecting greater disability. Sheehan D. The Anxiety Disease. New York, NY: Scribner; 1983.

The Profile of Mood States -Bipolar (POMS - Bi) scale measures moods and feelings primarily in clinical rather than nonclinical settings. It can help to determine an individual's psychiatric status for therapy, or be used to compare mood profiles associated with various personality disorders. It is also a useful instrument in identifying the effects of drug treatments.

The Post-Traumatic Cognitions Inventory (PTCI) is a 33- item scale, which is rated on a Likert- type scale ranging from 1 (totally disagree) to 7 (totally agree). Scale scores are formed for the three subscales, which show a high degree of intercorrelation (rs =.57 -.75).

The New Cognitions scale is a 6-item pilot scale, which is rated on a Likert-type scale ranging from 1 (not at all) to 4 (a lot). The scale is based on the Post Traumatic Growth Inventory (PTGI) from which items have been directly selected. New items were added to the scale as well.

The Yale-Brown Obsessive-Compulsive Scale (Y-BOCS) is the most widely used measure of OCD symptoms. It can be used as a self-report instrument or semi-structured interview, and has been demonstrated to be valid in OCD. The Y-BOCS exists in both an adult and a child version. This scale is not a diagnostic tool, but a reliable measure of symptom severity. The Y-BOCS has both a symptom checklist and a severity rating scale. The Y-BOCS severity scale consists of 10 questions: 5 about obsessions and 5 about compulsions. Response and non-response to treatment can be determined based on the Y-BOCS.

In other embodiments, determining whether a therapy disclosed herein is effective for treating a mood or anxiety disorder (e.g., PTSD) can be based on the self-evaluation of the patient being treated, e.g., if the patient reports that one or more symptoms of his or her mood and/or anxiety disorder has been treated following administration of a composition or formulation disclosed herein.

In certain embodiments, for PTSD, for example, the symptoms expected to be decreased following treatment with NPY (or NPY analog, active fragment or conjugate of NPY or NPY analog, or other NPY receptor ligand (including, e.g., Y1 receptor agonist and/or Y2 receptor antagonist)) include re-experiencing of the traumatic experience in the form of intrusive memories, nightmares, flashbacks, and emotional and physical reactions triggered by reminders of the trauma; distancing from others, decreased interest in activities and other people, numbing of feelings, and avoidance of trauma reminders; and hyperarousal symptoms, including disrupted sleep, irritability, hypervigilance, decreased concentration, and increased startle reflex.

Typically, the effects of treatment with a composition or formulation disclosed herein are observed by the patient and/or, e.g., the patient's physician, within a predetermined time frame from the time of administration. Typically, the predetermined time frame is starting within 2 hours and up to 24 hours following the administration of the composition or formulation. In other embodiments, the predetermined time frame is within 0.5 hours, 1 hour, 2.5 hours, 3 hours, 3.5 hours, 4 hours, 4.5 hours, 5 hours, 5.5 hours, 6 hours, 6.5 hours, 7 hours, 7.5 hours, 8 hours, 8.5 hours, 9 hours, 9.5 hours, 10 hours, 10.5 hours, 11 hours, 11.5 hours, 12 hours, 18 hours, 24 hours, 48 hours, or 72 hours following administration of the composition or formulation.

In yet other embodiments, the predetermined time frame is within 1 day, 2 days, 3 days, 4 days, 5 days, 6 days, 7 days, or 14 days following administration of the composition or formulation.

In still other embodiments, the predetermined time frame is within 1 minute, 2 minutes, 3 minutes, 4 minutes, 5 minutes, 6 minutes, 7 minutes, 8 minutes, 9 minutes, 10 minutes, 15 minutes, 20 minutes, 25 minutes, or 30 minutes following administration of the composition or formulation.

### EXAMPLES

The present disclosure is described further below in working examples which are intended to further describe the invention without limiting the scope therein.

### Example 1: NPY Dosage Study for Safety

This example demonstrates that a dosage of 200 nmol NPY (corresponding to 12 µg/kg NPY) was safe in patients with symptomatic PTSD. NPY was formulated in 0.9% USP-grade sterile saline.

A randomized, double-blinded, placebo-controlled crossover study was conducted in a medication-free, symptomatic PTSD group. Three (3) patients that had been diagnosed with PTSD and were not taking any medications were treated with an intranasal dose of 200 nmol (corresponding to 12 ng/kg). NPY administered using MAD Nasal™ device (LMA Company, San Diego, CA). No drug-related adverse effects and no dose-limiting toxicities (DLTs) were observed. DLT is defined as (1) Any serious adverse event with causality of at least "Possibly" or any moderate or severe adverse event with causality of at least "Possibly" based on FDA guidelines and the Mount Sinai School of Medicine Adverse Event tracking form and/or (2) The event that the patient's systolic or diastolic blood pressure (BP) or heart rate (HR) changes by greater than 20% relative to baseline values (baseline is defined by vital sign readings prior to the study drug administration).

### Example 2: Dose Escalation Studies

Dose escalation studies are conducted to test the safety and efficacy for treating PTSD of increasing doses of intranasally administered NPY, including 0.005 mg/kg, .01 mg/kg, .05 mg/kg and 1.0 mg/kg.

PTSD subjects are enrolled based on the traditional "3 + 3" dose escalation design. The dose escalation rules (see Table 2 below) proceed as follows, escalating in cohorts of 3-6 patients per dose level (see, Simon, R., B. Freidlin, et al. (1997). "Accelerated titration designs for phase I clinical trials in oncology." J Natl Cancer Inst 89(15): 1138-1147). Three patients are treated at the current dose level. If at least 2 patients are observed to have dose-limiting toxicity (DLT), the prior dose level is defined as the maximum tolerable dose (MTD) (unless only 3 patients have been treated at that level, in which case it is the tentative MTD). If 0 of the 3 patients are observed to have DLT, the dose level is escalated one step for the next cohort of 3 patients, and the process continues as above. If exactly 1 of the 3 patients treated show DLT, 3 additional patients are treated at the current dose level. If none of these additional 3 patients show DLT, the dose level is escalated for the next cohort of 3 patients, and the process continues as above; otherwise, the prior dose level is defined as the MTD (unless only 3 patients have been treated at that level, in which case it is the tentative MTD). A tentative MTD becomes final when a total of 6 patients are treated with less than 2 showing DLT.

### Example 3: Pilot Study of Efficacy of NPY for Treatment of PTSD

This study tests the efficacy in human patients of 0.005 mg/kg, .01 mg/kg, .05 mg/kg and 1.0 mg/kg NPY for the treatment of PTSD. At each dosage level, 3 study subjects are randomized to receive one NPY and one placebo (saline) intranasal administration on 2 study sessions that are one week apart. At each study session, patients are administered several clinical measures before and after the intranasal administration. Each study session will take about 3-4 hours. At the start of each study session, an intravenous catheter (in the forearm) is placed for sampling of plasma.

At the screen all participants rcccivc:
1). Psychiatric interviews and questionnaires:
   - Structured Clinical Interview for DSM-IV Axis I Disorders (SCID-1);
   - Clinician-Administered PTSD Scale (CAPS);
   - Beck Depression Inventory (Second Edition) (BDI-II);
   - Beck Anxiety Inventory (BAI);
   - Impact of Event Scale - Revised (IES-R);
   - Impact of Event Scale - Revised - Rapid (IES-R-Rapid);
   - State-Trait Anxiety Inventory - Trait (STAI-Trait);
   - State-Trait Anxiety Inventory - State (STAI-State);
   - Visual Analogy Mood/Anxiety Scale (VAS); and
   - Patient Rated Inventory of Side Effects (PRISE).
2). Medical assessments:
   - Medical history (Hx);
   - Physical examination (PE);
   - Vital signs (VS);
   - Electrocardiography (ECG);
   - Nasal endoscopy (NE) (Required only when subjects report a history of nasal conditions);
   - Laboratory tests;
      - Blood tests;
         ∘ Complete blood count with differential and platelets ("CBC w/ diff & plts");
         ∘ Comprehensive metabolic panel (CMP);
         ∘ Direct bilirubin (DB);
         ∘ Thyroid-stimulating hormone (TSH);
         ∘ Hepatitis B surface antigen (HBsAg);
         ∘ Hepatitis C Anti-Hepatitis C virus (HCV) antibody;
      - Urine tests;
         ∘ Drug abuse screen (Utox);
         ∘ Urinalysis (UA); and
         ∘ Pregnancy (women of childbearing potential).

The study investigators review the psychiatric interviews and medical assessments and identify eligible participants.

### 3). Trauma script

PTSD subjects usually present low symptoms at the study sessions. In order to better investigate drug efficacy, individualized trauma scripts are used as a symptom provocation method, which is a widely used method that has been shown to successfully induce anxiety and has minimal risk (see, Pitman, R. K., S. P. Orr, et al. (1987). "Psychophysiologic assessment of posttraumatic stress disorder imagery in Vietnam combat veterans." Archives of general psychiatry 44(11): 970-975; Bremner, J. D., M. Narayan, et al. (1999). "Neural correlates of memories of childhood sexual abuse in women with and without posttraumatic stress disorder." The American journal of psychiatry 156(11): 1787-1795). During the screening process, subjects describe one or two of their most traumatic experiences in writing on a script preparation form and then to select from a list of bodily responses that they remember accompanied the experiences. The drafts are reviewed by the study team and a final version of two scripts is composed approximately 30-50 seconds in duration. The two scripts are recorded in a neutral voice and are played separately at the two study sessions.

### 4). Medication washout

Psychotropic medications are not allowed throughout the study. If patients are taking psychotropic medications at screening, they must be tapered off over approximately 1 week and then be drug-free for a minimum of 1 week prior to study session 1. Patients who experience withdrawal symptoms from the medication taper may have this period extended. The monitoring of the tapering or "washout" is conducted by the patient's prescribing physician, with consultation from the study physician investigators. Study physician investigators actively participate in this process only if a patient no longer has an active treatment relationship with her/his prescribing physician.

### Visit 2: Study Session 1

Participants are randomized to receive one NPY and one placebo (saline) intranasal administration in 2 separate study sessions (Study Session 1 and 2). The procedures for Study Session 1 are listed in Table 3 below:

**Table 2. Dose Escalation Rules**

| | |
|---|---|
| **Outcome:** # DLT^{#} / # Pts | **Action:** Escalate, suspend, or halt dose escalation |
| 0 DLT out of 3 patients | Escalate dose for next cohort of 3 patients |
| 1 DLT out of 3 patients | Treat next cohort of 3 patients at the same dose |
| ≥ 2 DLT out of 3 patients | Halt dose escalation: treat total of 6 patients at previous dose to determine MTD* |
| 1 DLT out of 6 patients | Escalate dose for next cohort of 3 patients |
| ≥ 2 DLT out of 6 patients | Halt dose escalation: treat total of 6 patients at previous dose to determine MTD* |

| | |
|---|---|
| Table legend: *MTD (Maximum Tolerable Dose) --- the highest dose for which no more than 1 of the 6 treated patients exhibits DLT; ^{#}DLT (Dose-limiting Toxicity) is defined as 1). Any serious adverse event with causality of at least "Possibly" or any moderate or severe adverse event with causality of at least "Possibly" based on FDA guidelines and MSSM Adverse Event tracking form, or 2). The event that the patient's systolic or diastolic blood pressure (BP) or heart rate (HR) changes by greater than 20% relative to baseline values (baseline is defined by vital sign readings prior to the study drug administration). | |

It is expected that all of the dosages of NPY tested are safely administered intranasally.

**Table 3: Study Session 1 Procedure**

| **Time** | **Procedures** |
|---|---|
| 09:00am-10:00am | 1. Admission (confirm nil per os (NPO) since the night before) |
| | 2. Take vital signs upon arrival |
| | 3. Insertion of intravenous catheter |
| | 4. Questionnaires: |
| | - PRISE |
| | - BAI |
| | - Appetite scale |
| | - STAI-State |
| | - VAS |
| | - IES-R-Rapid |
| | 5. Plasma sampling |
| | 6. Take vital signs |
| 10:00am-10:20am | 1. Intranasal administration of NPY or placebo |
| | 2. Play trauma script |
| 10:20am (Omin) | 1. Questionnaires: |
| | - BAI |
| | - Appetite scale |
| | - STAI-State |
| | - VAS |
| | - IES-R-Rapid |
| | 2. Plasma sampling |
| | 3. Take vital signs |
| 10:50am (+30min) | 1. Questionnaires: |
| | - BAI |
| | - Appetite scale |
| | - STAI-State |
| | - VAS |
| | - IES-R-Rapid |
| | 2. Plasma sampling |
| | 3. Take vital signs |
| 11:20am (+60min) | 1. Questionnaires: |
| | - BAI |
| | - Appetite scale |
| | - STAI-State |
| | - VAS |
| | - IES-R-Rapid |
| | 2. Plasma sampling |
| | 3. Take vital signs |
| 11:50am (+90min) | 1. Questionnaires: |
| | - BAI |
| | - Appetite scale |
| | - STAI-State |
| | - VAS |
| | - IES-R-Rapid |
| | 2. Plasma sampling |
| | 3. Take vital signs |
| 12:20pm (+120min) | 1. Questionnaires: |
| | - BAI |
| | - Appetite scale |
| | - STAI-State |
| | - VAS |
| | - IES-R-Rapid |
| | - PRISE |
| | 2. Plasma sampling |
| | 3. Take vital signs |
| | 4. Remove intravenous catheter |
| | 5. Discharge home if no symptoms are present |

### a). Plasma sampling

For each plasma sampling 6 ml blood will be drawn. Blood is spun immediately and plasma is collected and stored in a freezer for measurement of plasma NPY concentration. Plasma NPY centration is measured using radioimmunoassay (see, Allen, R., J. Boublik, et al. (1991). "Neuropeptide Y radioimmunoassay: characterization and application." Clin Exp Pharmacol Physiol 18(12): 825-833).

### b). Dosage of NPY

The doses of NPY for intranasal administration are 0.005 mg/kg, .01 mg/kg, .05 mg/kg and 1.0 mg/kg, dissolved in sterile 0.9% USP-grade saline. The placebo is 0.9% USP-grade saline without NPY.

### c). Intranasal administration of NPY or placebo

Participants receive an intranasal administration of either NPY or placebo. Intranasal administration is given by an atomizer (MAD Nasal™, LMA) connected to a syringe. NPY is administered with multiple sprays in both nostrils until all of intended amount of drug is administered.

### d). Vital signs

Vital signs are measured repeatedly during the study session to ensure safety.

The following psychiatric interview tools and questionnaires are used:
1. The Structured Clinical Interview for DSM-IV Axis I Disorders (SCID-I) (First, Spitzer et al. 2002, *supra)* is a standard clinician-rated interview to assess the presence of Axis I psychiatric disorders according to DSM-IV criteria.
2. The Clinician-Administered PTSD Scale (CAPS) (Blake, Weathers et al. 1995, *supra)* is a 30-item structured interview that corresponds to the DSM-IV criteria for PTSD. The CAPS can be used to make a current (past month) or lifetime diagnosis of PTSD or to assess symptoms over the past week.
3. The Patient Rated Inventory of Side Effects (PRISE) Adverse Event Visit Checklist is a clinician-administered questionnaire used to qualify side effects by identifying and evaluating the tolerability of each symptom.
4. The Beck Depression Inventory (Second Edition) (BDI-II) (Beck, Steer et al. 1996, *supra*) is a 21-item self-report instrument intended to assess the existence and severity of symptoms of depression. The questionnaire is designed for individuals aged 13 and over, and is composed of items relating to symptoms of depression such as hopelessness and irritability, cognitions such as guilt or feelings of being punished, as well as physical symptoms such as fatigue, weight loss, and lack of interest in sex.
5. The Beck Anxiety Inventory (BAI) (Beck and Steer 1993, *supra)* is a 21-question multiple-choice self-report inventory that is used for measuring the severity of an individual's anxiety. The BAI consists of twenty-one questions about how the subject has been feeling in the last week, expressed as common symptoms of anxiety (such as numbness, hot and cold sweats, or feelings of dread).
6. The Appetite Scale (Bond and Lader 1974, *supra)* is a visual analog scale to measure level of hunger.
7. The Impact of Event Scale - Revised (IES-R) (Weiss and Marmar 1996, *supra)* is a 22-item self-report measure that assesses subjective distress caused by traumatic events.
8. The Impact of Event Scale - Revised - Rapid (IES-R-Rapid) is a 14-item self-report measure that assesses immediate subject distress caused by traumatic events.
9. The Visual Analogue Scales (VAS) (Bond and Lader 1974, *supra)* are used to assess subjective state changes. They are 100-mm horizontal lines marked proportionately to the perceived intensity of the subjective experience (0=not at all, to 10=extremely) for the following states: anxious, depressed, drowsy, high, hungry, and nauseous.
10. The State-Trait Anxiety Inventory (STAI) (Spielberger 1985, *supra)* is a self-report instrument that differentiates between the temporary condition of state anxiety and the longstanding quality of trait anxiety. With a fifth grade reading level, the STAI is suitable for individuals who are 15 years old and older.
11. The MSSM Adverse Event tracking form is a clinician-administered form for systematic documentation of adverse events with FDA-recommended rating scales of severity and causality.

### Visit 3: Study Session 2

The procedures of Study Session 2 are the same as Study Session 1. Participants receive either NPY or placebo at this session, whichever they do not receive at Session 1.
Visit 4: Study Exit

Participants come for a final assessment. The study physicians evaluate any possible side effects or symptoms that might result from the study. The participants complete the following rating scales at this visit:
- PRISE; and
- Adverse Event tracking form.

It is expected that participants treated with 0.005 mg/kg, .01 mg/kg, .05 mg/kg and 1.0 mg/kg NPY experience a reduction in one or more symptoms of PTSD.

### Example 4: Study of Efficacy of NPY for

### Treatment of Major Depressive Disorder

This study tests the efficacy in human patients of 0.005 mg/kg, .01 mg/kg, .05 mg/kg and 1.0 mg/kg NPY for the treatment of Major Depressive Disorder. At each dosage level, 3 study subjects are randomized to receive one NPY and one placebo (saline) intranasal administration on 2 study sessions that are one week apart. At each study session, patients are administered several clinical measures before and after the intranasal administration. Each study session will take about 3-4 hours. At the start of each study session, an intravenous catheter (in the forearm) is placed for sampling of plasma.

Each study session is carried out as in Example 3. The following clinical measures, which are described above, are used to assess the symptoms of Major Depressive Disorder before and after treatment, as well as side effects of treatment:
- Structured Clinical Interview for DSM-IV Axis I Disorders (SCID-I);
- Beck Depression Inventory (Second Edition) (BDI-II);
- The Montgomery-Asberg Depression Rating Scale (MADRS);
- Beck Anxiety Inventory (BAI);
- State-Trait Anxiety Inventory - Trait (STAI-Trait);
- State-Trait Anxiety Inventory - State (STAI-State);
- Visual Analogy Mood/Anxiety Scale (VAS); and
- Patient Rated Inventory of Side Effects (PRISE).

It is expected that participants treated with 0.005 mg/kg, .01 mg/kg, .05 mg/kg and 1.0 mg/kg NPY experience a reduction in one or more symptoms of Major Depressive Disorder.

### Example 5: Study of Efficacy of NPY for

### Treatment of Obsessive-Compulsive Disorder

This study tests the efficacy in human patients of 0.005 mg/kg, .01 mg/kg, .05 mg/kg and 1.0 mg/kg NPY for the treatment of Obsessive-Compulsive Disorder (OCD). At each dosage level, 3 study subjects are randomized to receive one NPY and one placebo (saline) intranasal administration on 2 study sessions that are one week apart. At each study session, patients are administered several clinical measures before and after the intranasal administration. Each study session will take about 3-4 hours. At the start of each study session, an intravenous catheter (in the forearm) is placed for sampling of plasma.

Each study session is carried out as in Example 3. Clinical measures for assessing symptoms of OCD before and after treatment, as well as any side effects of treatment, that can be used include:
(1) Yale-Brown Obsessive-Compulsive Scale (Y-BOCS):
   The Yale-Brown Obsessive-Compulsive Scale (Y-BOCS) is the most widely used measure of OCD symptoms. It can be used as a self-report instrument or semi-structured interview, and has been demonstrated to be valid in OCD. The Y-BOCS exists in both an adult and a child version. This scale is not a diagnostic tool, but a reliable measure of symptom severity. The Y-BOCS has both a symptom checklist and a severity rating scale. The Y-BOCS severity scale consists of 10 questions: 5 about obsessions and 5 about compulsions. Response and non-response to treatment is based on the Y-BOCS; optionally,
(2) The Structured Clinical Interview for DSM-IV Axis I Disorders, Patient Edition (SCID-P); and, optionally,
(3) Patient Rated Inventory of Side Effects (PRISE).

It is expected that participants treated with 0.005 mg/kg, .01 mg/kg, .05 mg/kg and 1.0 mg/kg NPY experience a reduction in one or more symptoms of OCD.

### Example 6: Total Dosage Studies using NPY for Treatment of PTSD

A study is performed as described in Example 3, above, with the following total doses per day of NPY: 100 mg/day, 80 mg/day, 60 mg/day, 40 mg/day, 20 mg/day, 10 mg/day and 1 mg/day, or placebo.

Some treatment groups receive the total dose divided into two, three, of four administrations, and some treatment groups receive a single administration of the total dose.

It is expected that participants treated with a total dose of 100 mg/day, 80 mg/day, 60 mg/day, 40 mg/day, 20 mg/day, 10 mg/day and 1 mg/day NPY experience a reduction in one or more symptoms of PTSD.

### Example 7: Total Dosage Studies using NPY for Treatment of Major Depressive Disorder

A study is performed as described in Example 4, above, with the following total doses per day of NPY: 100 mg/day, 80 mg/day, 60 mg/day, 40 mg/day, 20 mg/day, 10 mg/day and 1 mg/day, or placebo.

Some treatment groups receive the total dose divided into two, three, or four administrations, and some treatment groups receive a single administration of the total dose.

It is expected that participants treated with a total dose of 100 mg/day, 80 mg/day, 60 mg/day, 40 mg/day, 20 mg/day, 10 mg/day and 1 mg/day NPY experience a reduction in one or more symptoms of Major Depressive Disorder.

### Example 8: Total Dosage Studies using NPY for Treatment of OCD

A study is performed as described in Example 5, above, with the following total doses per day of NPY: 100 mg/day, 80 mg/day, 60 mg/day, 40 mg/day, 20 mg/day, 10 mg/day and 1 mg/day, or placebo.

Some treatment groups receive the total dose divided into two, three, or four administrations, and some treatment groups receive a single administration of the total dose.

It is expected that participants treated with a total dose of 100 mg/day, 80 mg/day, 60 mg/day, 40 mg/day, 20 mg/day, 10 mg/day and 1 mg/day NPY experience a reduction in one or more symptoms of OCD.

The present disclosure is not to be limited in scope by the specific embodiments described herein. Indeed, various modifications of the invention in addition to those described herein will become apparent to those skilled in the art from the foregoing description. Such modifications arc intended to fall within the scope of the appended claims.

It is further to be understood that all values are approximate, and are provided for description.

Patents, patent applications, publications, product descriptions, and protocols are cited throughout this application, the disclosures of which are incorporated herein by reference in their entireties for all purposes.

The invention is further embodied by the following items:
1. A method for treating a human patient for a mood or anxiety disorder which comprises intranasally administering to a human patient in need of such treatment a composition comprising a therapeutically effective dose of neuropeptide Y (NPY) for reducing or eliminating one or more symptoms of the mood or anxiety disorder.
2. The method of item 1, wherein the therapeutically effective dose is in the range of about 1 mg to about 20 mg of NPY.
3. The method of item 1 or 2, which comprises administering the therapeutically effective dose to the patient at least once a day.
4. The method of item 1, wherein the dose is in the range of about 1 mg to about 100 mg, about 1 mg to about 90 mg, about 1 mg to about 80 mg, about 1 mg to about 70 mg, about 1 mg to about 60 mg, about 1 mg to about 50 mg, about 1 mg to about 40 mg, about 1 mg to about 30 mg, about 1 mg to about 20 mg, about 1 mg to about 10 mg, or about 1 mg to about 5 mg.
5. The method of item 1 or 3, which comprises administering a total dose to the patient of between about 1 mg and about 100 mg/day of NPY.
6. The method of item 5, wherein the total dose is in the range of about 1 mg to about 100 mg, about 1 mg to about 90 mg, about 1 mg to about 80 mg, about 1 mg to about 70 mg, about 1 mg to about 60 mg, about 1 mg to about 50 mg, about 1 mg to about 40 mg, about 1 mg to about 30 mg, about 1 mg to about 20 mg, about 1 mg to about 10 mg, or about 1 mg to about 5 mg.
7. The method of item 1, wherein the method comprises administering to the subject a single dose of the composition.
8. The method of item 1, wherein the method comprises administering to the subject multiple doses of the composition.
9. The method of item 8, wherein the method comprises administering to the subject from 1 to 4 doses of the composition per day.
10. The method of item 1, wherein the intranasal administration comprises atomizing the dose administered to the patient.
11. The method of item 10, wherein the atomized dose is administered to the patient as one or more intranasal sprays.
12. The method of item 9, wherein the multiple doses are administered on separate days.
13. The method of item 9, wherein at least two of the multiple doses are administered on the same day.
14. The method of item 1, which comprises co-administering the composition and a second active agent together or at spaced-apart time intervals in a combination therapy.
15. The method of item 14, wherein the second active agent comprises an antidepressant.
16. The method of any of the preceding items, wherein the composition further comprises a pharmaceutically acceptable carrier.
17. The method of item 16, wherein the pharmaceutically acceptable carrier is sterile saline.
18. The method of any of items 1-15, wherein the disorder in posttraumatic stress disorder (PTSD).
19. A pharmaceutical formulation comprising: (a) at least about 0.005 mg/kg NPY and (b), a pharmaceutically acceptable carrier, wherein the pharmaceutical formulation is suitable for intranasal administration.
20. A pharmaceutical formulation comprising: (a) at least about 0.01 mg/kg NPY and (b), a pharmaceutically acceptable carrier, wherein the pharmaceutical formulation is suitable for intranasal administration.
21. A method of treating a mood or anxiety disorder comprising administering to a human patient in need of such treatment the pharmaceutical formulation of item 18 or 19.
22. A method for treating a human patient for a mood or anxiety disorder which comprises intranasally administering to a human patient in need of such treatment a composition comprising a therapeutically effective amount of a Y1 receptor agonist for reducing or eliminating the symptoms of the mood or anxiety disorder, wherein the therapeutically effective amount is a dosage range of about 0.005 milligrams per kilogram of body weight of the patient (mg/kg) to about 1 mg/kg.
23. A method for treating a human patient for a mood or anxiety disorder which comprises intranasally administering to a human patient in need of such treatment a composition comprising a therapeutically effective amount of a Y2 receptor antagonist for reducing or eliminating the symptoms of the mood or anxiety disorder, wherein the therapeutically effective amount is a dosage range of about 0.005 milligrams per kilogram of body weight of the patient (mg/kg) to about 1 mg/kg.
24. An intranasal dosage form comprising: (a) NPY and (b), a pharmaceutically acceptable diluent.
25. The dosage form of item 24, comprising NPY in a dosage range from about 0.005 milligrams per kilogram of body weight (mg/kg) to about 2 mg/kg, from about 0.005 mg/kg to about 1.5 mg/kg, from about 0.005 mg/kg to about 1 mg/kg, from about 0.01 mg/kg to about 1.0 mg/kg, from about 0.05 mg/kg to about 1.0 mg/kg, or from about 0.05 mg/kg to about 0.5 mg/kg.
26. The dosage form of item 24, comprising NPY in a dosage range from about 0.05 mg to about 200 mg, from about 0.05 mg to about 150 mg, from about 0.05 mg to about 125 mg, from about 0.5 mg to about 100 mg, from about 1 mg to about 100 mg, from about 1 mg to about 50 mg, from about 1 mg to about 40 mg, from about 1 mg to about 35 mg, from about 1 mg to about 30 mg, from about 1 mg to about 25 mg, from about 1 mg to about 20 mg, from about 1 mg to about 15 mg, from about 1 mg to about 10 mg, or from about from about 1 mg to about 5 mg.
27. The intranasal dosage form of item 24 wherein the diluent is a member selected from the group consisting of sterile water, saline solution, buffered saline and dextrose solution.
28. The dosage form of item 24 or 25, further comprising a pharmaceutically acceptable excipient.
29. An aerosol formulation comprising NPY or an NPY analog and an aerosol propellant.
30. The aerosol dosage form of item 29 wherein NPY or NPY analog comprises a dry powder.
31. The aerosol dosage form of item 30 further comprising a bulking agent.

## Claims

1. A composition comprising a therapeutically effective dose of 1 mg to 100 mg of neuropeptide Y (NPY) formulated as an aerosol for administration directly to the olfactory region of the nasal cavity for use in reducing or eliminating one or more symptoms of major depressive disorder (MDD).

2. The composition for use according to claim 1, wherein the dose is in the range of 1 mg to 90 mg, 1 mg to 80 mg, 1 mg to 70 mg, 1 mg to 60 mg, 1 mg to 50 mg, 1 mg to 40 mg, 1 mg to 30 mg, 1 mg to 20 mg, 1 mg to 10 mg, or 1 mg to 5 mg.

3. The composition for use according to claim 1, including a second active agent for administration at the same time or at spaced-apart time intervals in a combination therapy.

4. The composition for use according to claim 3, wherein the second active agent comprises an antidepressant.

5. The composition for use according to any of the preceding claims, wherein the composition further comprises a pharmaceutically acceptable carrier.

6. A pharmaceutical formulation comprising: (a) at least 0.005 mg/kg NPY and (b) a pharmaceutically acceptable carrier and formulated as an aerosol, wherein the pharmaceutical formulation is formulated for administration directly to the olfactory region of the nasal cavity.

7. The pharmaceutical formulation of claim 6, comprising at least 0.01 mg/kg NPY.

8. A composition comprising a therapeutically effective amount of a Y1 receptor agonist and a pharmaceutically acceptable diluent for use in reducing or eliminating the symptoms of major depressive disorder (MDD), wherein the therapeutically effective amount is a dosage range of 0.005 milligrams per kilogram of body weight of the patient (mg/kg) to 1 mg/kg.

9. A composition comprising a therapeutically effective amount of a Y2 receptor antagonist and a pharmaceutically acceptable diluent for use in reducing or eliminating the symptoms of major depressive disorder (MDD), wherein the therapeutically effective amount is a dosage range of 0.005 milligrams per kilogram of body weight of the patient (mg/kg) to 1 mg/kg.

10. An aerosol intranasal dosage form for administration directly to the olfactory region of the nasal cavity comprising: (a) 1 mg to 100 mg of NPY and (b) a pharmaceutically acceptable diluent.

11. The dosage form of claim 10, comprising NPY in a dosage range from about 0.005 milligrams per kilogram of body weight (mg/kg) to 2 mg/kg, from 0.005 mg/kg to 1.5 mg/kg, from 0.005 mg/kg to 1 mg/kg, from 0.01 mg/kg to 1.0 mg/kg, from 0.05 mg/kg to 1.0 mg/kg, or from 0.05 mg/kg to 0.5 mg/kg.

12. The dosage form of claim 10, comprising NPY in a dosage range from 1 mg to 50 mg, from 1 mg to 40 mg, from 1 mg to 35 mg, from 1 mg to 30 mg, from 1 mg to 25 mg, from 1 mg to 20 mg, from 1 mg to 15 mg, from 1 mg to 10 mg, or from 1 mg to 5 mg.

13. An aerosol formulation comprising: a) 1 mg to 100 mg of NPY or an NPY analog having at least one amino acid addition, insertion, substitution or deletion in the amino acid sequence of NPY and b) an aerosol propellant, contained in a device that delivers the formulation directly to the olfactory region in the roof of the nasal cavity.

14. The aerosol formulation of claim 13 wherein the device delivers the formulation as a spray.

15. The composition of claim 1 including a vasoconstrictor.


SEQUENCE LISTING

<110> Icahn School of Medicine at Mount Sinai

<120> Treatment of Mood and Anxiety Disorders

<130> EP103338I-HV200pau

<140> not yet assigned
<141> 2014-05-13

<150> EP14798340.7
<151> 2014-05-13

<150> PCT/US2014/037907
<151> 2014-05-13

<150> 61/822,619
<151> 2013-05-13

<160> 5

<170> FastSEQ for Windows Version 4.0

<210> 1
<211> 36
<212> PRT
<213> Artificial Sequence

<220>
<223> peptide analog

<400> 1
Tyr Pro Ser Lys Pro Asp Asn Pro Gly Glu Asp Ala Pro Ala Glu Asp
 1               5                   10                  15
Met Ala Arg Tyr Tyr Ser Ala Leu Arg His Tyr Ile Asn Leu Ile Thr
            20                  25                  30
Arg Gln Arg Tyr
        35

<210> 2
<211> 36
<212> PRT
<213> Artificial Sequence

<220>
<223> peptide analog

<400> 2
Tyr Pro Ser Lys Pro Asp Asn Pro Gly Glu Asp Ala Pro Ala Glu Asp
 1               5                   10                  15
Met Ala Arg Tyr Tyr Ser Ala Leu Arg His Tyr Ile Asn Leu Leu Thr
            20                  25                  30
Arg Pro Arg Tyr
        35

<210> 3
<211> 36
<212> PRT
<213> Artificial Sequence

<220>
<223> peptide analog

<400> 3
Tyr Pro Ser Lys Pro Asp Asn Pro Gly Glu Asp Ala Pro Ala Glu Asp
1               5                   10                  15
Leu Ala Arg Tyr Tyr Ser Ala Leu Arg His Tyr Ile Asn Leu Leu Thr
            20                  25                  30
Arg Pro Arg Tyr
        35


<210> 4
<211> 36
<212> PRT
<213> Artificial Sequence

<220>
<223> peptide analog

<400> 4
Tyr Pro Ser Lys Pro Asp Asn Pro Gly Glu Asp Ala Pro Ala Glu Asp
1               5                   10                  15
Leu Ala Arg Tyr Tyr Ser Ala Leu Arg His Tyr Ile Asn Leu Ile Thr
            20                  25                  30
Arg Pro Arg Tyr
        35


<210> 5
<211> 15
<212> PRT
<213> Artificial Sequence

<220>
<223> peptide analog

<400> 5
Tyr Lys Gly Arg Glu Tyr Ile Lys Leu Ile Thr Arg Pro Arg Tyr
1               5                   10                  15